# EUROPÄISCHE PATENTANMELDUNG

(11) **EP 1 038 868 A2**
(43) Veröffentlichungstag der Anmeldung: **27.09.2000**
(21) Anmeldenummer: 00104463.5
(22) Anmeldetag: 07.03.2000
(51) Int. Cl.: C07D 213/75, C07C 311/21, C07D 213/73, C07D 239/46, C07D 243/08, C07D 213/74, A61K 31/18, A61P 31/12, C07D 295/20, C07D 207/12, C07D 307/52, C07D 211/74, C07D 491/10, C07D 209/14, C07D 211/58, C07D 211/46, C07D 211/22, C07D 211/32, C07D 211/62, C07D 277/04, C07D 273/00, C07D 277/46, C07D 231/40, C07D 471/04

(54) **Sulfonamide als antivirale Mittel**

(30) Priorität: 20.03.1999 DE 19912638
(71) Anmelder: Bayer Aktiengesellschaft, 51368 Leverkusen (DE)
(72) Erfinder: Reefschläger, Jürgen, Dr., 42111 Wuppertal (DE); Bender, Wolfgang, Dr., 42113 Wuppertal (DE); Eckenberg, Peter, Dr., 42115 Wuppertal (DE); Goldmann, Siegfried, Dr., 42327 Wuppertal (DE); Härter, Michael, Dr., 51375 Leverkusen (DE); Hallenberger, Sabine, Dr., 42109 Wuppertal (DE); Trappe, Jörg, Dr., 20052 Monza (IT); Weber, Olaf, Dr., 42489 Wülfrath (DE)

(57) **Zusammenfassung**

Die vorliegende Erfindung betrifft neue Verbindungen der allgemeinen Formel (I): 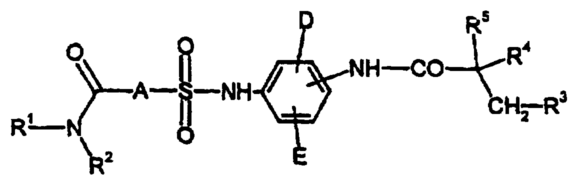 worin
- A: für einen zweiwertigen (C₆-C₁₀)Arylrest oder einen zweiwertigen 5- bis 10-gliedrigen Heteroarylrest steht die gegebenenfalls substituiert sein können durch ein bis drei Substituenten, die aus der Gruppe ausgewählt werden, die aus (C₁-C₃)Alkyl, Hydroxy oder Halogenatomen besteht,
die als Arzneimittel geeignet sind, Verfahren zu ihrer Herstellung und ihre Verwendung als Arzneimittel, insbesondere als antivirale Mittel.

## Beschreibung

Die vorliegende Erfindung betrifft neue Verbindungen, die als Arzneimittel geeignet sind, Verfahren zu ihrer Herstellung und ihre Verwendung als Arzneimittel, insbesondere als antivirale Mittel.

α,β-Naphthyl verknüpfte Phenylsulfonamide sind überwiegend aus phototechnischen Publikationen bekannt [vgl. hierzu JP-06 122 669-A2, EP-684 515-A1; JP-59 174 836-A2, DE-2 902 074, US-3 925 347, US-4 035 401, US-3 622 603, US-3 482 971, EP-284 130].

Die WO 90/09 787 offenbart Sulfonamide als Radio- oder Chemosensibilisierungsmittel und ihre Verwendung bei der Behandlung von Tumoren.

Außerdem ist die Verbindung N-[4-[[[5-(Dimethylamino)-1-naphthalenyl]sulfonyl]amino]phenyl]-acetamid bekannt (J. Inst. Chem. (India) (1976), 48, Pt 6, 280-5).

Die Erfindung betrifft Verbindungen der allgemeinen Formel ( I ): 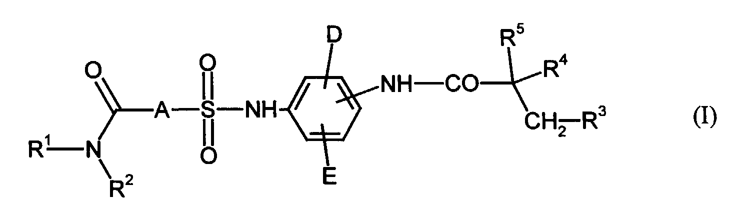 worin
- R¹: für Wasserstoff oder eine geradkettige, verzweigte oder cyclische, gesättigte oder ungesättigte Kohlenwasserstoffgruppe mit 1 bis 14 Kohlenstoffatomen steht, worin gegebenfalls ein bis zwei Kohlenstoffatome in der Kette durch O oder S ersetzt sein können, und die durch ein bis drei Substituenten substituiert sein kann, die aus der Gruppe ausgewählt werden, die aus Hydroxy, Cyano, -N(R⁶)R⁷, (C₁-C₆)Alkoxycarbonyl, (C₆-C₁₀)Aryl und 5- bis 10-gliedrigem Heteroaryl besteht, wobei (C₆-C₁₀)Aryl und 5- bis 10-gliedriges Heteroaryl ihrerseits durch ein bis drei Substituenten substituiert sein können, die aus Hydroxy, (C₁-C₆)Alkyl, (C₁-C₆)Alkoxy, (C₁-C₆)-Alkoxycarbonyl, Halogen und Halogen(C₁-C₆)alkyl ausgewählt werden,
und worin R⁶ und R⁷ gleich oder verschieden sind und für Wasserstoff, (C₁-C₆)Alkoxycarbonyl oder eine geradkettige, verzweigte oder cyclische, gesättigte oder ungesättigte Kohlenwasserstoffgruppe mit 1 bis 10 Kohlenstoffatomen, die gegebenfalls mit Di(C₁-C₆)Alkylamino substituiert ist, stehen, oder gemeinsam mit dem Stickstoffatom, an das sie gebunden sind, einen 3- bis 8-gliedrigen heterocyclischen Ring bilden, der gegebenfalls durch 1 bis 3 (C₁-C₆)Alkylgruppen substituiert sein kann und/oder ein oder zwei weitere Heteroatome ausgewählt aus O, S oder N enthalten kann,
oder
- R¹: für (C₆-C₁₀)Aryl oder 5- bis 10-gliedriges Heteroaryl steht, die gegebenenfalls durch ein bis drei Substituenten substituiert sein können, die aus der Gruppe ausgewählt werden, die besteht aus (C₁-C₆)Alkyl, Hydroxy, (C₁-C₆)Alkoxycarbonyl, Halogen und Amino, oder
- R¹: für (5- bis 10-gliedriges Heteroaryl)amino oder (C₆-C₁₀)Aryl(C₁-C₆)alkylamino steht,
- R²: für (C₁-C₆)Alkoxy, (C₁-C₆)Alkoxycarbonyl, Aminocarbonyl steht oder eine der oben genannten Bedeutungen von R¹ aufweisen kann und von dieser Bedeutung gleich oder verschieden sein kann,
oder
- R¹ und R²: gemeinsam mit dem Stickstoffatom, an das sie gebunden sind einen gesättigten oder ungesättigten Ring bilden mit bis zu 14 C-Atomen, dessen Ringkohlenstoffatome gegebenfalls durch 1 bis 4 Reste ausgewählt aus der Gruppe, die aus O , S, S=O, C=O und N-R⁸ besteht, ersetzt sein können, und wobei der Ring gegebenfalls substituiert sein kann durch ein bis drei Substituenten, ausgewählt aus der Gruppe, die besteht aus Hydroxy, (C₁-C₆)-Alkyl, Benzyl, Hydroxy(C₁-C₆)alkyl, Phenylamino, Benzoyl, (C₁-C₆)Acyl, (C₁ -C₆)Alkoxycarbonyl, Aminocarbonyl, Mono- oder Di(C₁-C₆)alkylaminocarbonyl, Morpholinocarbonyl, Phenylaminocarbonyl, (C₁-C₆)Alkoxycarbonyl(C₁-C₆)alkyl, (C₁-C₆)Alkoxyaminocarbonyl, (C₁ -C₆)-Alkoxycarbonylamino, 1,3-Dioxolan-2,2-diyl, Piperidyl, Piperidino, Imino,
worin R⁸ für Wasserstoff, eine geradkettige, verzweigte oder cyclische, gesättigte oder ungesättigte Kohlenwasserstoffgruppe mit bis zu 10 Kohlenstoffatomen steht, die gegebenfalls durch 1 bis 3 Reste ausgewählt aus der Gruppe, die aus Hydroxy, Pyrrolidinyl, Pyridyl, Phenyl, Halogenphenyl, Halogen(C₁-C₃)alkylphenyl und (C₁-C₆)-Alkoxycarbonyl besteht, substituiert sein kann,
oder R⁸ für (C₁-C₆)Alkoxycarbonyl, (C₆-C₁₀)Aryl oder 5- bis 10-gliedriges Heteroaryl steht, wobei (C₆-C₁₀)Aryl und 5- bis 10-gliedriges Heteroaryl ihrerseits durch ein bis drei Substituenten substituiert sein können, die aus der Gruppe bestehend aus Halogen, Hydroxy, (C₁-C₆)Alkoxy, Nitro oder Fluor(C₁-C₃)alkyl besteht,
- R³: für Wasserstoff, Halogen, Hydroxy, (C₁-C₆)Acyl, Benzoyl oder (C₁-C₆)Acyloxy steht,
- R⁴ und R⁵: unabhängig voneinander eine (C₁-C₃)Alkylgruppe sind oder gemeinsam mit dem Kohlenstoffatom, an das sie gebunden sind einen 3-bis 6-gliedrigen carbocyclischen Ring bilden,
- A: für einen zweiwertigen (C₆-C₁₀)Arylrest oder einen zweiwertigen 5-bis 10-gliedrigen Heteroarylrest steht die gegebenenfalls substituiert sein können durch ein bis drei Substituenten, die aus der Gruppe ausgewählt werden, die aus (C₁-C₃)Alkyl, Hydroxy oder Halogenatomen besteht, und
- D und E: gleich oder verschieden sind und für Wasserstoff, Fluor, Chlor, Nitro, Cyano, Hydroxy oder für (C₁-C₃)Alkyl (C₁-C₃)Alkoxy oder (C₁-C₃)-Alkoxycarbonyl stehen,
sowie deren pharmazeutische verträgliche Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfaßt.

Eine geradkettige, verzweigte oder cyclische, gesättigte oder ungesättigte Kohlenwasserstoffgruppe mit 1 bis 14 Kohlenstoffatomen schließt im Rahmen der Erfindung beispielsweise ein, einen geradkettigen oder verzweigten Alkylrest mit 1 bis 14 Kohlenstoffatomen, einen geradkettigen oder verzweigten Alkenylrest mit 2 bis 14 Kohlenstoffatomen, einen geradkettigen oder verzweigten Alkinylrest mit 2 bis 14 Kohlenstoffatomen sowie einen gesättigten oder ungesättigten cyclischen Kohlenwasserstoffrest mit 3 bis 6 Kohlenstoffatomen in der Definition von R¹. Desweiteren schließen Reste in denen 1 bis 2 Kohlenstoffatome der obengenannten Kohlenwasserstoffgruppen in der Kette durch O oder S ersetzt sein können, beispielsweise (C₁-C₆)Alkoxyalkyl, (C₁-C₆)Alkylthioalkyl-Reste ein.

Bezüglich der geradkettigen oder verzweigten Alkylgruppe mit 1 bis 14 Kohlenstoffatomen seien beispielsweise genannt: Methyl, Ethyl, Propyl, Isopropyl, Butyl, Isobutyl, sek.-Butyl, Pentyl, Isopentyl, hexyl, Isohexyl, Heptyl, Isoheptyl, Octyl, Nonyl, Decyl, Undecyl und Dodecyl.

Als geradkettige oder verzweigte Alkenylgruppe mit 2 bis 14 Kohlenstoffatomen seien beispielsweise genannt: Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl.

Als Alkinylgruppe mit 2 bis 14 Kohlenstoffatomen können geradkettige oder verzweigte Reste erwähnt werden, die eine oder mehrere C≡C-Gruppen aufweisen. Bevorzugt sind Alkinylreste mit 2 bis 8 Kohlenstoffatomen und beispielsweise seien Acetylenyl, 2-Butinyl, 2-Pentinyl und 2-Hexinyl genannt.

Die oben erwähnte Cycloalkylgruppe mit 3 bis 6 Kohlenstoffatomen schließt beispielsweise ein Cyclopropyl, Cyclopentyl und Cyclohexyl. Bevorzugt ist Cyclopropyl.

Bevorzugt ist die geradkettige, verzweigte oder cyclische, gesättigte oder ungesättigte Kohlenwasserstoffgruppe mit 1 bis 14 Kohlenstoffatomen in der Definition von R¹ eine geradkettige Alkylgruppe mit 1 bis 12 Kohlenstoffatomen oder eine (C₁-C₆)Alkoxy(C₁-C₆)alkylgruppe.

Bezüglich der (C₁-C₆)Alkoxygruppe sei auf die unten erwähnte Definition verwiesen. (C₁-C₆)Alkyl steht im Rahmen der Erfindung im allgemeinen für geradkettige oder verzweigtkettige Kohlenwasserstoffreste mit 1 bis 6 Kohlenstoffatomen, und diesbezüglich sei auf die beispielhaft genannten Gruppen in den Erläuterungen zur Alkylgruppe mit 1 bis 14 Kohlenstoffatomen oben verwiesen.

Die (C₁-C₆)Alkoxygruppe, wie sie in der vorliegenden Erfindung verwendet wird, und wie sie auch in den Definitionen (C₁-C₆)Alkoxycarbonyl, (C₁-C₆)Alkoxycarbonyl(C₁-C₆)alkyl und (C₁-C₆)Alkoxycarbonylamino verwendet wird, schließt beispielsweise geradkettige oder verzweigtkettige Alkoxygruppen mit 1 bis 6 Kohlenstoffatomen ein, besonders bevorzugt Alkoxygruppen mit 1 bis 4 Kohlenstoffatomen, noch bevorzugter Alkoxygruppen mit 1 bis 3 Kohlenstoffatomen. Beispielsweise können erwähnt werden Methoxy, Ethoxy, Propoxy, Isopropoxy, Butoxy, Isobutoxy, Pentoxy, Isopentoxy, Hexoxy und Isohexoxy. Bevorzugt ist Methoxy, Ethoxy und Propoxy.

Die geradkettige, verzweigte oder cyclische, gesättigte oder ungesättigte Kohlenwasserstoffgruppe mit 1 bis 10 Kohlenstoffatomen in der Definition von R⁶ und R⁷ schließt beispielsweise die oben für die Definition der geradkettigen, verzweigtkettigen oder cyclischen, gesättigten oder ungesättigten Kohlenwasserstoffgruppen mit 1 bis 14 Kohlenstoffatomen in der Definition von R¹ genannten Gruppen mit 1 bis 10 Kohlenstoffatomen ein. Bevorzugt ist hier eine geradkettige oder verzweigtkettige (C₁-C₆)Alkylgruppe.

(C₁-C₆)Alkylamino schließt im Rahmen der Erfindung die Alkylaminogruppen ein, deren Alkylgruppen 1 bis 6 Kohlenstoffatome aufweisen. Dabei kann es sich um symmetrische oder unsymmetrische Alkylaminogruppen handeln, wie beispielsweise Dimethylamino, Diethylamino, Methylethylamino usw.

3- bis 8-gliedrige Ringe, die R⁶ und R⁷ gemeinsam mit dem Stickstoffatom an die sie gebunden sind, bilden können, schließen beispielsweise Pyrrolidin, Piperidin, Morpholin etc.

(C₆-C₁₀)Aryl steht im Rahmen der Erfindung für einen aromatischen Rest mit 6 bis 10 Kohlenstoffatomen. Bevorzugte Arylreste sind Phenyl und Naphthyl. 5- bis 10-gliedriges Heteroaryl steht im Rahmen der Erfindung für 5- bis 10-gliedrige Heteroatome enthaltende Ringe, die 1 bis 4 Heteroatome enthalten können, die ausgewählt werden aus O, S und N und schließen beispielsweise ein Pyridyl, Furyl, Thienyl, Pyrolyl, Imidazolyl, Pyrazolyl, Pyrazinyl, Pyrimidinyl, Pyridazinyl, Indolizinyl, Indolyl, Benzo[b]thienyl, Indazolyl, Chinolyl, Isochinolyl, Naphthyridinyl, Chinazolinyl, etc.

Bevorzugt sind 6- oder 10-gliedrige aromatische Heterocyclen, die gegebenenfalls wie in den Ansprüchen definiert, substituiert sein können.

Halogen schließt im Rahmen der Erfindung Fluor, Chlor, Brom und Iod ein. Bevorzugt sind Chlor oder Fluor.

Halogen(C₁-C₆)alkyl schließt im Rahmen der Erfindung (C₁-C₆)Alkylgruppen ein, wie oben definiert, die durch 1 bis 3 Halogenatome, bevorzugt Fluor oder Chlor, substituiert sein können wie beispielsweise Monofluoralkylgruppen mit 1 bis 3 Kohlenstoffatomen, Monochloralkylgruppen mit 1 bis 3 Kohlenstoffatomen, sowie Difluoralkylgruppen mit 1 bis 6 Kohlenstoffatomen, Dichloralkylgruppen mit 1 bis 6 Kohlenstoffatomen sowie Trifluor- bzw. Trichloralkylgruppen mit 1 bis 6 Kohlenstoffatomen. Bezüglich der Alkylgruppen sei auf die obigen Erläuterungen verwiesen.

Besonders bevorzugt sind Fluormethyl, Chlormethyl sowie Trifluormethyl.

5- bis 10-gliedriges Heteroarylamino schließt Amino ein, das durch eine der oben erwähnten 5 bis 10-gliedrigen Heteroarylreste substituiert ist. Bevorzugt ist hier beispielsweise Pyridylamino.

Bezüglich (C₆-C₁₀)Aryl(C₁-C₆)alkylamino sei auf die oben erwähnten Definitionen für (C₆-C₁₀)Aryl und (C₁-C₆)Alkyl verwiesen. Ein bevorzugtes Beispiel stellt hier Benzylamino dar.

Gesättigte oder ungesättigte Ringe mit bis zu 14 Kohlenstoffatomen, R¹ und R² gemeinsam mit dem Stickstoffatom an die sie gebunden sind in denen 1 bis 4 Ringkohlenstoffatome durch 1 bis 4 Reste ausgewählt aus der Gruppe, die aus O, S, S=O, C=O und N-R⁸ besteht, ersetzt sein können, schließen beispielsweise im Rahmen der Erfindung ein: Morpholino, Piperazinyl, Piperidyl, das gegebenenfalls ein- oder zweifach ungesättigt sein kann, 1-Azacycloheptan, 1,4-Diazacycloheptan, Pyrrolidinyl, Thiomorpholino (1,4-Thiazinan-4-yl), 1-Azacyclooctan-1-yl, 1-Azacycloheptan- 1-yl, 1,3-Thiazolidin-3-yl, 1-Aza-4,7,10,13-tetraoxocyclopentadecan-1-yl und Dihydropyrrol-1-yl.

Bezüglich der geradkettigen, verzweigtkettigen oder cyclischen, gesättigten oder ungesättigten Kohlenwasserstoffe mit bis zu 10 Kohlenstoffatomen in der Definition für R⁸ sei auf die oben erwähnte Definition der geradkettigen, verzweigten oder cyclischen, gesättigten oder ungesättigten Kohlenwasserstoffgruppen mit 1 bis 10 Kohlenstoffatomen in der Definition von R⁶ und R⁷ verwiesen.

Fluor(C₁-C₃)alkyl schließt im Rahmen der Erfindung Alkylgruppen mit 1 bis 3 Kohlenstoffatomen mit bis zu 3 Fluoratomen ein wie z.B. Trifluormethyl.

Hydroxy(C₁-C₆)alkyl schließt im Rahmen der Erfindung die oben erwähnten geradkettigen oder verzweigtkettigen Alkylgruppen mit 1 bis 6 Kohlenstoffatomen ein, die durch 1 bis 3 Hydroxygruppen substituiert sein können. Bevorzugt ist Hydroxymethyl.

(C₁-C₆)Acyl sowie (C₁-C₆)Acyl in der Definition (C₁-C₆)Acyloxy steht im Rahmen der Erfindung für geradkettiges oder verzweigtkettiges Alkanoyl mit 1 bis 6 Kohlenstoffatomen. Beispielsweise seien erwähnt: Formyl, Acetyl, Propanoyl, Isopropanoyl, Butanoyl, Isobutanoyl, Pentanoyl und Hexanoyl.

In einer bevorzugten Ausführungform schließt die Erfindung Verbindungen der obigen allgemeinen Formel (I) ein, worin:
- R¹: für Wasserstoff, eine (C₃-C₆)-Cycloalkylgruppe, eine (C₂-C₆)-Alkenylgruppe oder eine geradkettige oder verzweigtkettige (C₁-C₁₂)-Alkylgruppe steht, in der gegebenenfalls ein Kettenkohlenstoffatom durch Sauerstoff ersetzt ist, und die gegebenenfalls durch 1 bis 3 Substituenten substituiert sein kann, die aus der Gruppe ausgewählt werden, die aus Hydroxy, Cyano, -N-(R⁶)R⁷, (C₁-C₆)-Alkoxycarbonyl, (C₆-C₁₀)-Aryl und 5- bis 10-gliedrigem Heteroaryl besteht, wobei (C₆-C₁₀)-Aryl und 5- bis 10-gliedriges Heteroaryl ihrerseits durch 1 bis 3 Substituenten substituiert sein können, die ausgewählt werden aus (C₁-C₃)-Alkoxy und (C₁-C₃)-Alkyl, und worin
- R⁶ und R⁷: gleich oder verschieden sind und für Wasserstoff, (C₁-C₆)-Alkoxycarbonyl und/oder (C₁-C₆)Alkyl, das gegebenfalls durch Di(C₁-C₆)Alkylamino substituiert ist, stehen, oder gemeinsam mit dem Stickstoffatom, an das sie gebunden sind, einen 3- bis 6-gliedrigen heterocyclischen Ring bilden, der gegebenenfalls ein zusätzliches Sauerstoffatom enthalten kann,
oder
- R¹: für Phenyl oder 5- bis 10-gliedriges Heteroaryl, das 1 bis 2 Heteroatome ausgewählt aus Stickstoff und Schwefel enthalten kann, steht, die jeweils gegebenenfalls durch 1 bis 2 Substituenten, ausgewählt aus Amino, (C₁-C₃)-Alkyl, Hydroxy, (C₁-C₄)-Alkoxycarbonyl und Halogen, substituiert sein können, oder
- R¹: für (5- bis 6-gliedriges) Heteroarylamino oder (C₆-C₁₀)Aryl(C₁-C₃)alkylamino steht,
- R²: für (C₁-C₆)Alkyl, (C₁-C₆)Alkoxy, und Aminocarbonyl steht, oder die oben genannte Bedeutung von R¹ aufweisen kann und von dieser Bedeutung gleich oder verschieden sein kann, oder
- R¹ und R²: gemeinsam mit dem Stickstoffatom, an das sie gebunden sind einen gesättigten oder ungesättigten Ring mit 4 bis 14 Kohlenstoffatomen bilden, dessen Ringkohlenstoffatome gegebenenfalls durch 1 bis 4 Reste ausgewählt aus der Gruppe, die aus O, S, S=O, C=O und N-R⁸ besteht, ersetzt sein können, und wobei der Ring gegebenenfalls durch 1 bis 3 Substituenten substituiert sein kann, die aus der Gruppe ausgewählt werden, die besteht aus Imino, Hydroxy, (C₁-C₃)Alkyl, 1,3-Dioxolan-2,2-diyl, (C₁-C₃)Alkoxycarbonyl, (C₁-C₆)Alkoxycarbonylamino, Benzyl, Phenylamino, Piperidyl, Hydroxy-(C₁-C₃)alkyl, Benzoyl, Aminocarbonyl, (C₁-C₃)Acyl, (C₁-C₆)Alkylaminocarbonyl, Morpholinocarbonyl und Phenylaminocarbonyl besteht,
und worin
- R⁸: für Wasserstoff, eine geradkettige oder verzweigtkettige (C₁-C₆)Alkylgruppe, die gegebenenfalls durch 1 bis 2 Substituenten ausgewählt werden kann, die aus der Gruppe substituiert sein kann, die aus Phenyl, Hydroxy, Halogenphenyl, Pyrrolidinyl und (C₁-C₃)Alkoxycarbonyl besteht, oder
- R⁸: für (C₃-C₆)Cycloalkyl oder Phenyl steht, das gegebenenfalls substituiert sein kann mit 1 bis 3 Substituenten, die aus der Gruppe ausgewählt werden, die besteht aus Halogen, Hydroxy, (C₁-C₃)Alkoxy, Nitro und Trifluormethyl, oder
- R⁸: für Pyridyl oder (C₁-C₆)Alkoxycarbonyl steht,
- R³: für Wasserstoff, Halogen, Hydroxy oder (C₁-C₃)Acyloxy steht,
und
- R⁴ und R⁵: jeweils unabhängig für eine (C₁-C₃)Alkylgruppe stehen,
- A: für einen Naphthalindiylrest oder einen Phenylenrest steht, die gegebenenfalls durch eine (C₁-C₃)Alkylgruppe, Halogen oder Hydroxy substituiert sein können, und
- D und E: für Wasserstoff stehen,
sowie deren pharmazeutisch verträglichen Salze.

In einer weiteren bevorzugten Ausführungsform schließt die Erfindung Verbindungen der obigen Formel (I) ein, worin
- R¹: für Wasserstoff, Cyclopropyl, (C₂-C₆)Alkenyl, (C₁-C₆)Alkoxy(C₁-C₆)alkyl oder eine geradkettige oder verzweigtkettige (C₁-C₁₂)Alkylgruppe steht, die gegebenenfalls durch 1 bis 3 Substituenten substituiert sein kann, die aus der Gruppe ausgewählt werden die aus Hydroxy, Cyano, -N(R⁶)R⁷, Phenyl, das gegebenenfalls durch (C₁-C₃)Alkoxy substituiert sein kann, Pyridyl, das gegebenenfalls durch (C₁-C₃)Alkyl substituiert sein kann, Furyl, Indolyl und (C₁-C₄)Alkoxycarbonyl besteht,
worin
- R⁶ und R⁷: gleich oder verschieden sein können und ausgewählt werden aus Wasserstoff, (C₁-C₆)Alkoxycarbonyl und (C₁-C₃)Alkyl, das gegebenenfalls durch Di(C₁-C₃)Alkylamino substituiert sein kann, oder
- R⁶ und R⁷: gemeinsam mit dem Stickstoffatom, an das sie gebunden sind Morpholino oder Piperidino bilden, das gegebenenfalls durch 1 bis 2 (C₁-C₃)Alkylgruppen substituiert sein kann,
oder
- R¹: für Phenyl steht, das gegebenenfalls durch 1 bis 3 Substituenten substituiert sein kann, die ausgewählt werden aus der Gruppe die aus Hydroxy, (C₁-C₄)Alkoxycarbonyl, Halogen und (C₁-C₃)Alkyl besteht, oder für Isochinolinyl, Pyridyl, das gegebenenfalls durch (C₁-C₃)Alkyl oder Amino substituiert sein kann, Pyrimidinyl, das gegebenenfalls durch Hydroxy substituiert sein kann, Thiazolyl, Pyrazolyl oder für Naphthyridinyl steht, oder
- R¹: für Pyridylamino oder Benzylamino steht, und
- R²: die gleiche Bedeutung wie die von R¹, wie zuvor definiert, aufweisen kann, und mit dieser Bedeutung gleich oder verschieden sein kann oder
- R²: für (C₁-C₃)Alkoxy oder Aminocarbonyl stehen kann,
oder
- R¹ und R²: gemeinsam mit dem Stickstoffatom, an das sie gebunden sind, einen Ring bilden, der ausgewählt wird aus der Gruppe die aus Morpholino, das gegebenenfalls durch 1 bis 3 (C₁-C₃)Alkylgruppen substituiert sein kann,
Thiomorpholino, das gegebenenfalls ein Sauerstoffatom am Schwefel tragen kann,
Thiazolidinyl,
1-Azacyclopent-3-en-1-yl,
1-Aza-4,7,10,13-tetraoxacyclopentadecan-1-yl,
einem 5- bis 8-gliedrigen gesättigten Ring, in dem gegebenenfalls ein Ringkohlenstoffatom durch eine Gruppe der Formel -NR⁸ ersetzt sein kann, worin
R⁸ ausgewählt wird aus Wasserstoff, gegebenfalls durch 1 bis 3 Substituenten ausgewählt aus Halogen, Hydroxy, (C₁ -C₃)Alkoxy, Trifluormethyl und/oder Nitro, substituiertem Phenyl, Halogenphenyl, gegebenfalls durch 1 bis 2 Halogenphenylgruppen, Phenyl und/oder Pyrrolidinyl substituiertem (C₁-C₃)Alkyl, Cyclopropyl, Hydroxy(C₁-C₃)alkyl, Pyridyl und (C₁-C₄)Alkoxycarbonyl,
und der gegebenfalls durch 1 bis 2 Substituenten ausgewählt aus (C₁-C₃)Alkyl, Hydroxy(C₁-C₃)alkyl, Hydroxy, 1,3-Dioxolan-2,2-diyl, Oxo, (C₁-C₄)Alkoxycarbonyl, (C₁-C₄)Alkoxycarbonylamino, Benzyl, Phenylamino, Piperidino, Benzoyl, Aminocarbonyl, (C₁-C₃)Acyl, (C₁-C₆)Alkylaminocarbonyl, Morpholinocarbonyl und Phenylaminocarbonyl substituiert sein kann,
und einem Rest der Formel: 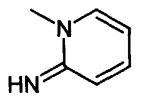 besteht,
- R³: für Wasserstoff, Chlor, Fluor, Acetyloxy, Hydroxy steht,
- R⁴ und R⁵: für Methyl stehen,
- A: für Naphthalin-1,5-diyl, Naphthalin-1,6-diyl oder 1,3-Phenylen steht, das gegebenenfalls durch Hydroxy, Chlor, Fluor oder (C₁-C₃)Alkyl substituiert sein kann, und worin
- D und E: für Wasserstoff stehen, und deren Salze.

In einer weiteren bevorzugten Ausführungsform schließt die Erfindung Verbindungen der folgenden Formel (I 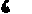 ) ein:

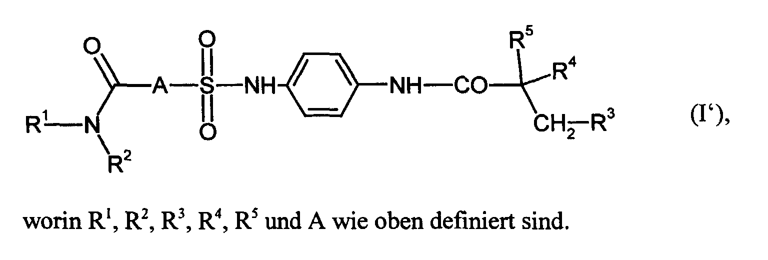

In einer weiteren bevorzugten Ausführungsform schließt die Erfindung Verbindungen der Formel (I) oder (I 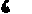 ) ein, worin A Naphthalindiyl ist.

In einer weiteren bevorzugten Ausführungsform schließt die Erfindung Verbindungen der Formel (I) oder (I 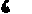 ) ein, worin R¹ Wasserstoff ist und R² ausgewählt wird aus Pyridyl, Phenyl, Hydroxyphenyl oder (C₁-C₄)Alkoxycarbonylphenyl, oder R¹ und R² gemeinsam mit dem Stickstoffatom, an das sie gebunden sind, einen Ring der Formel: 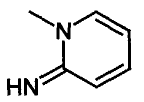 bilden, oder für Thiomorpholino, 4-Oxopiperidino, 4-N-(Cyclopropyl)piperazin-1-yl,Piperidino, 1-Azacyclooctan-1-yl, 1-Azacycloheptan-1-yl oder 1,3-Thiazolidin-1-yl stehen.

In einer weiteren bevorzugten Ausführungsform schließt die Erfindung Verbindungen der Formel (I) oder (I 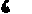 ) ein, worin
- R³: für Wasserstoff oder Halogen steht
- R⁴ und R⁵: jeweils für eine Methylgruppe stehen,
- A: für einen Phenylen oder Naphthalindiyl-Rest steht, und
- D und E: für Wasserstoff stehen.

Die Erfindung betrifft weiter ein Verfahren zur Herstellung von Verbindungen der allgemeinen Formel (I) oder (I 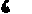 ) (Die Verbindungen der allgemeinen Formel (I) schließen die Verbindungen der allgemeinen Formel (I 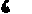 ) ein), dadurch gekennzeichnet, daß man Verbindungen der allgemeinen Formel (II) 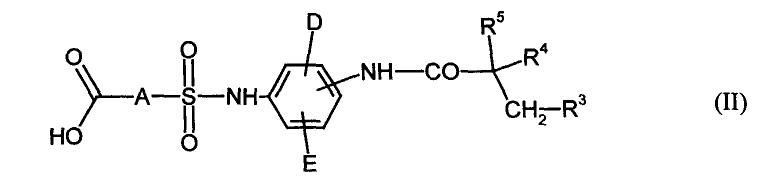
worin die Substituenten die obengenannte Bedeutung aufweisen, gegebenenfalls nach Überführung in das Säurechlorid oder nach Aktivierung nach literaturbekannten Verfahren, mit Aminen der allgemeinen Formel (III) 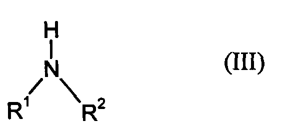 umsetzt, oder
Verbindungen der allgemeinen Formel (IV) 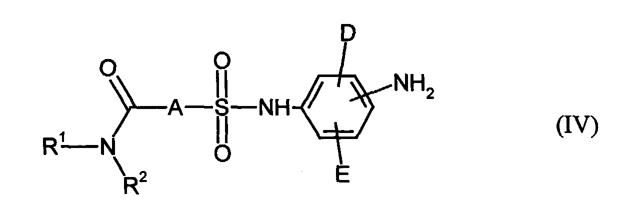
worin die Substituenten die obengenannte Bedeutung besitzen, mit Carbonsäurechloriden der allgemeinen Formel (V) umsetzt 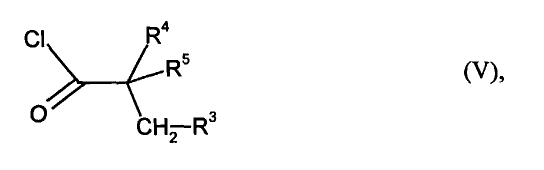
worin die Substituenten die obengenannte Bedeutung besitzen.

Das erfindungsgemäße Verfahren kann durch folgendes Formelschema beispielhaft erläutert werden:

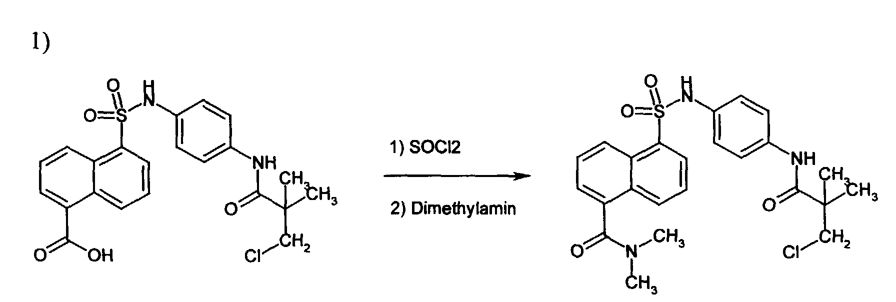

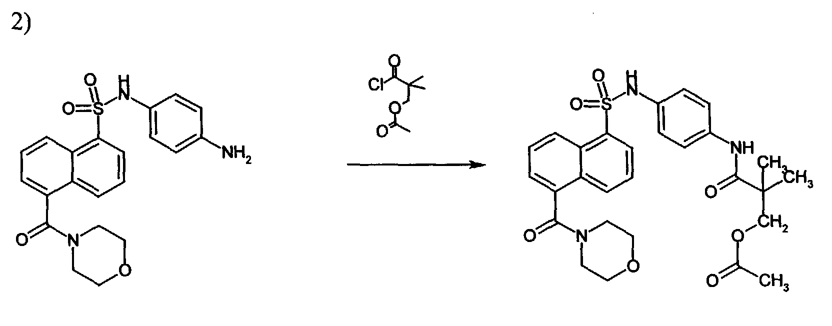

Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Pyridin und Dioxan.

Als Basen eignen sich organische Amine, insbesondere Trialkyl(C₁-C₆)amine wie beispielsweise Triethylamin oder Heterocyclen wie Pyridin, Methylpiperidin, Piperidin oder N-Methylmorpholin. Bevorzugt sind Pyridin, Triethylamin und N-Methylmorpholin.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0°C bis 100°C, vorzugsweise bei 0°C bis 30°C und bei Normaldruck durchgeführt.

Die Erfindung betrifft weiterhin Verbindungen der Formel (I) bzw. (I') zur Verwendung als Arzneimittel.

Die Erfindung betrifft weiterhin eine pharmazeutische Zusammensetzung, die eine Verbindung der allgemeinen Formel (I) bzw. (I 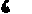 ) in Mischung mit mindestens einem pharmazeutisch verträglichen Träger oder Exzipienten umfaßt.

Die Erfindung betrifft weiterhin die Verwendung der Verbindung der allgemeinen Formel (I) bzw. (I 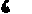 ) zur Herstellung eines Arzneimittels.

Die Erfindung betrifft weiterhin die Verwendung einer Verbindung der allgemeinen Formel (I) bzw. (I 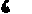 ) zur Herstellung eines Arzneimittels zur Behandlung von viralen Infektionen, insbesondere Infektionen durch Cytomegalieviren.

Die erfindungsgemäßen Verbindungen der allgemeinen Formeln (I) zeigen ein nicht vorhersehbares überraschendes Wirkspektrum. Sie zeigen eine antivirale Wirkung gegenüber Vertretern der Gruppe der Herpes viridae, besonders gegenüber dem humanen Cytomegalievirus (HCMV). Sie eignen sich somit zur Behandlung und Prophylaxe von Erkrankungen, die durch Herpes-Viren, insbesondere Erkrankungen, die durch humanes Cytomegalievirus (HCMV) hervorgerufen werden.

Die Anti-HCMV-Wirkung wurde in einem Screening-Testsystem in 96-Well-Mikrotiterplatten unter Zuhilfenahme von humanen embryonalen Lungenfibroblasten (HELF)-Zellkulturen bestimmt. Der Einfluß der Substanzen auf die Ausbreitung des cytopathogenen Effektes wurde im Vergleich zu der Referenzsubstanz Ganciclovir (Cymevene^{R}-Natrium), einem klinisch zugelassenen anti-HCMV-Chemotherapeutikum, bestimmt.

Die in DMSO (Dimethylsulfoxid) gelösten Substanzen (50 mM) werden auf Mikrotiterplatten (96-Well) in Doppelbestimmungen (4 Substanzen/Platte) untersucht. Toxische und cytostatische Substanzwirkungen werden dabei miterfaßt. Nach den entsprechenden Substanzverdünnungen (1:2) auf der Mikrotiterplatte wird eine Suspension von 50 HCMV-infizierten HELF-Zellen und 3 x 10⁴ nichtinfizierten HELF-Zellen in Eagle's MEM mit 10% fötalem Kälberserum in jedes Näpfchen gegeben, und die Platten bei 37°C in einem CO₂-Brutschrank über 6 Tage inkubiert. Nach dieser Zeit ist der Zellrasen in den substanzfreien Viruskontrollen, ausgehend von 50 infektiösen Zentren, durch den cytopathogenen Effekt des HCMV völlig zerstört (100% CPE). Nach einer Anfärbung mit Neutralrot und Fixierung mit Formalin / Methanol werden die Platten mit Hilfe eines Projektions-Mikroskopes (Plaque-Viewer) ausgewertet.

Die erfindungsgemäßen Verbindungen stellen somit wertvolle Wirkstoffe zur Behandlung und Prophylaxe von Erkrankungen dar, die durch humanes Cytomegalievirus ausgelöst werden. Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.

### In vivo-Wirkung

### Tiere

5 Wochen alte männliche Mäuse, Stamm NOD/LtSz-scid/j, wurden von einem kommerziellen Züchter (The Jackson Lab., Bar Harbor) bezogen. Die Tiere wurden unter sterilen Bedingungen (einschließlich Einstreu und Futter) in Isolatoren gehalten.

### Virus/Infektion

Murines Cytomegalievirus (MCMV), Stamm Smith, wurde in vivo (in BALB/c-Mäusen) passagiert und über eine fraktionierte Zentrifugation aufgereinigt. Der Titer wurde mit Hilfe eines Plaqueassays auf primären embryonalen Mäusefibroblasten untersucht. Die Infektion der Mäuse erfolgte mit einer Dosis von 5x10⁵ pfu in einem Gesamtvolumen von 0,2 ml intraperitoneal. Diese Dosis führt bei 100% der infizierten Tiere nach ca. 11 Tagen zum Tode.

### Behandlung/Auswertung

20 Stunden nach der Infektion wurden die Mäuse über einen Zeitraum von 8 Tagen zweimal täglich (morgens und abends) per os mit Substanz behandelt. Die Dosis betrug 25 mg/kg Körpermasse, das Applikationsvolumen 10 ml/kg Körpermasse. Die Formulierung der Substanzen erfolgte in Form einer 0,5%igen Tylosesuspension. 16 Stunden nach der letzten Substanzapplikation wurden die Tiere schmerzlos getötet und Speicheldrüse, Leber und Niere entnommen.

Aus 25 mg der Gewebe wurde über Phenol/Chloroform-Extraktion genomische DNA aufgereinigt. Die Quantifizierung der DNA erfolgte photometrisch und mit Hilfe der Formel OD₂₆₀x50=mg/ml.

Die Reinheit der DNA wurde über den Quotienten OD₂₆₀/OD₂₈₀ kontrolliert und die DNA anschließend mit Tris-EDTA pH = 8,0 eingestellt.

Die Quantifizierung der MCMV-DNA erfolgte mittels DNA-Dot-Blot-Hybridisierung. Als Sonde wurde ein Digoxygenin-gelabeltes (Boehringer-Mannheim, ebenfalls aufgeführte Puffer, wenn nicht anders beschrieben) 1,2 kb Fragment aus dem Bereich MCMV, Smith, HindIII J, verwendet. Die Detektion der Signale erfolgte mittels Chemolumineszenz. Dafür wurde die Membran für 3 Minuten in 1 x Digoxygenin-Waschpuffer 1 gewaschen. Im Anschluß wurden die Filter für 30 Minuten bei Raumtemperatur unter Schütteln in 1 x Digoxygenin Blockierungslösung inkubiert. Die Filter wurden danach für 30 Minuten in 20 ml/100 cm² Membran mit der Anti-DIG-Alkalische-Phosphatase-Konjugatlösung (1:20000 in 1 x Digoxygenin Blockierungslösung) inkubiert. 2 je 15 Minuten dauernde Waschschritte mit 1 x Digoxygenin-Waschpuffer schlossen sich an. Es folgten 5 Minuten Äquilibrierung der Filter in 1 x Digoxygenin-Detektionspuffer und die Detektion mittels 1 ml / 100 cm² Membranfläche 1:100 verdünnte CDP-Star-Lösung. Nach Ausstreichen der CDP-Star-Lösung und 5 minütiger Inkubation in einer dunklen Box erfolgte der Nachweis der Chemolumineszenz bzw. die Auswertung mittels Röntgenfilm (Kodak) oder Lumilmager (Boehringer Mannheim).

Alle Ergebnisse wurden statistisch gesichert (Varianzanalyse mittels Statistika; StatSoft Inc.).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hufslösemittel verwendet werden können.

Die Applikation erfolgt in üblicher Weise, vorzugsweise oral, parenteral oder topisch, insbesondere perlingual, intravenös oder intravital gegebenenfalls als Depot in einem Implantat.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschritten werden muß. Im Falle der Applikation größerer Mengen kann es empfehlenswert sein, diese in mehreren Einzelgaben über den Tag zu verteilen.

Gegebenenfalls kann es sinnvoll sein, die erfindungsgemäßen Verbindungen mit anderen Wirkstoffen zu kombinieren.

### Beispiele

### Ausgangsverbindungen:

### Beispiel I

### 5-(Chlorsulfonyl)-1-naphthalincarbonsäure

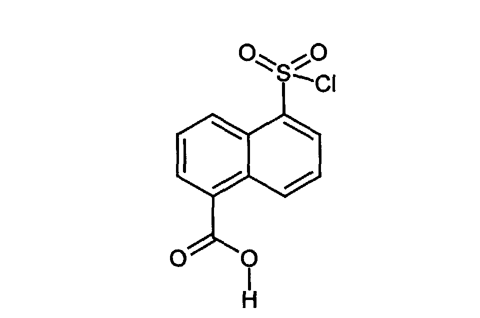

25 g 1-Naphthalincarbonsäuremethylester werden unter Eiskühlung tropfenweise mit 78 g Chlorsulfonsäure versetzt, wobei die Temperatur unter 20°C gehalten wird. Nach Rühren über Nacht bei Raumtemperatur wird mit 120 ml Methylenchlorid verdünnt und vorsichtig auf 800 g Eiswasser gegeben. Der weiße Niederschlag wird abgesaugt, mit kaltem Wasser gewaschen und sofort über Nacht im Vakuum über P₂O₅ getrocknet. Ausbeute 5,5 g (15%). (Substanz enthält noch geringe Mengen des Methylesters).
1H-NMR (DMSO-D6): 7,55(1H,dd,J_{1,2}=7-8Hz), 7,60 (1H,dd,J_{1,2}=7-8Hz), 8,05 (1H,d,J=7Hz), 8,10 (1H,d,J=7Hz), 8,85 (1H,d,J=8Hz), 9,25 (1H,d,J=8Hz), 14,1(COOH) ppm.

### Beispiel II

### 5-({4-[(2,2-Dimethylpropanoyl)amino]anilino}sulfonyl)-1-naphthalincarbonsäure

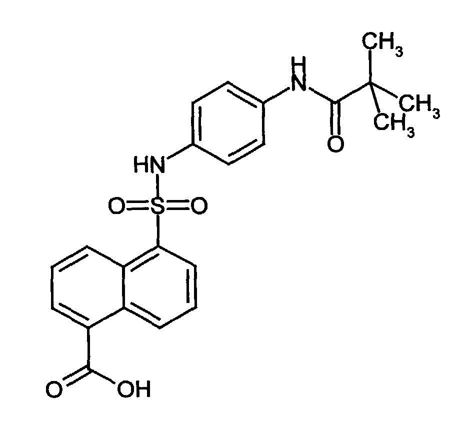

5,0 g (Chlorsulfonyl)-1-naphthalincarbonsäure (Beispiel I) werden in 50 ml wasserfreiem Dioxan gelöst und mit 4,8 g N-(4-Aminophenyl)-pivaloylamid (Lit.: Rajappa, Srinivasachari; Sreenivasan, Ramaswami; Khalwadekar, Asha, JRMPDM, J. Chem. Res. Miniprint, EN, 5, 1986, 1657-1675) versetzt. Nach 5 min bei RT werden 1,8 g Natriumhydrogencarbonat zugegeben und über Nacht am Rückfluß gekocht. Nach Abkühlung wird mit Essigester verdünnt und viermal mit 1N HCl ausgeschüttelt. Es wird über Natriumsulfat getrocknet und eingeengt. Nach Aufnehmen in Methylenchlorid Kristallbildung. (FP: 234°C), RF-Wert 0,22 (Acetonitril/Wasser=95/5).

### Beispiel III

### 5-({4-[(3-Chlor-2,2-dimethylpropanoyl)amino]anilino}sulfonyl)-1-naphthalincarbonsäure

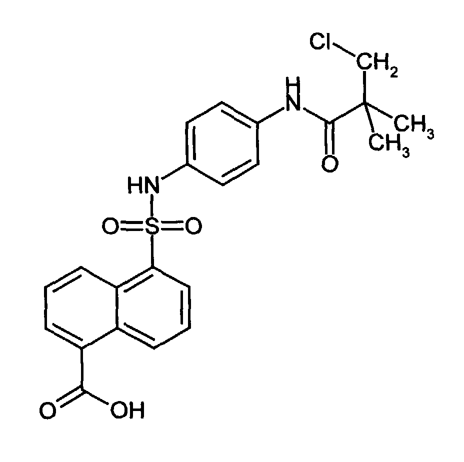

Die Herstellung erfolgt analog Beispiel II
FP: ab 120°C Zersetzung,
Rf: 0,21(Acetonitril/Wasser=95:5)

### Beispiel IV:

### 5-({4-[(3-Fluor-2,2-dimethylpropanoyl)amino]anilino}sulfonyl)-1-naphthalincarbonsäure

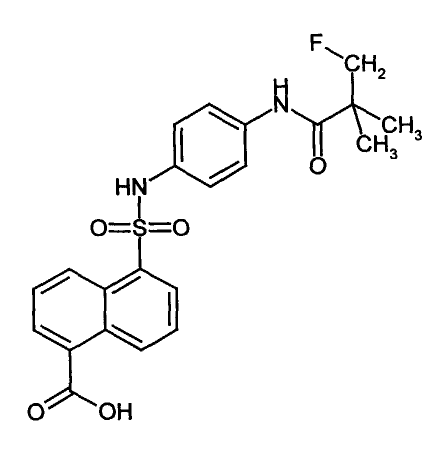

Die Herstellung erfolgt analog Beispiel II
Rf: 0,23(Acetonitril/Wasser=95:5)

### Beispiel V:

### 3-({4-[(3-Chlor-2,2-dimethylpropanoyl)amino]anilino}sulfonyl)-1-benzoesäure (38-8728) (38-8728)

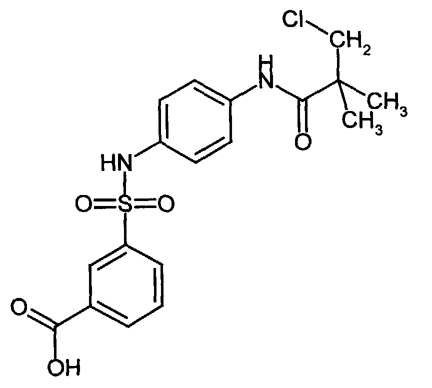

Die Darstellung erfolgt analog Beispiel II mit 3-Chlorsulfonyl-benzoesäure. Ausbeute 92 %.
1H-NMR(DMSO-D6): 1,25(s,6H), 3,8(s,2H), 7,0(d,J=10Hz,2H), 7,45(d,J=10Hz,2H), 7,65 (t,J=9Hz,1H), 7,9(t,1H), 8,1(d,J=9Hz,1H), 8,3(s,1H), 9,3(s,NH), 10,2(s,NH), 10,3 bis 12,5(-COOH) ppm

### Endprodukte

### Beispiel 1

### 5-({4-[(3-Chlor-2,2-dimethylpropanoyl)amino]anilino}sulfonyl)-N-butyl-1-naphthamid

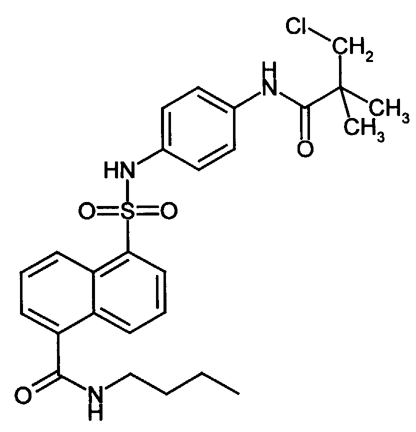

0,3 g 5-({4-[(3-Chlor-2,2-dimethylpropanoyl)amino]anilino} sulfonyl)-1-naphthalincarbonsäure (**Beispiel III**) werden mit 1 ml Thionylchlorid versetzt und am Rückfluß gekocht bis die Gasentwicklung beendet ist. Überschüssiges Thionylchlorid wird im Vakuum abgezogen, in CCl₄ aufgenommen und im Vakuum getrocknet. Der Rückstand wird in 1 ml trocknem THF gelöst mit 0,01 ml Butylamin und 0,5 ml Triethylamin versetzt und über Nacht bei RT gerührt. Nach Chromatographie erhält man 90 mg 5-({4-[(3-chloro-2,2-dimethylpropanoyl)amino]anilino} sulfonyl)-N-butyl-1-naphthamid.
RF-Wert: 0,18 (a)
1H-NMR (DMSO-D6): 0,9(t,J=7Hz,3H), 1,25(s,6H), 1,25-1,6(m,4H), 3,25(q,J=7Hz,2H), 3,8(s,2H), 7,0(d,J=8Hz,2H), 7,45(d,J=8Hz,2H), 7,6(t,J=8Hz,1H), 7,8(d,J=8Hz,1H), 8,25(s,1H), 8,7(t,NH), 9,3(s,NH), 10,2(s,NH) ppm.

### Beispiel 2

### 5-({4-[(3-Chlor-2,2-dimethylpropanoyl)amino]anilino}sulfonyl)-N,N-dimethyl-1-naphthamid

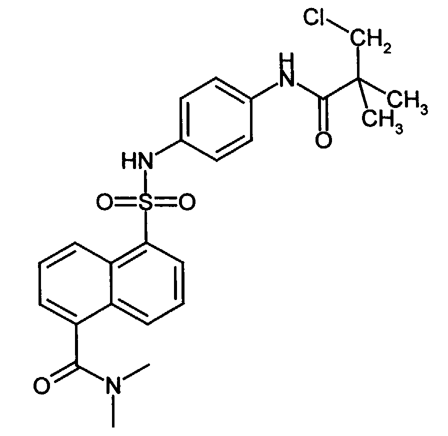

0,1 g 5-({4-[(3-Chlor-2,2-dimethylpropanoyl)amino]anilino}sulfonyl)-1-naphthalincarbonsäure (**Beispiel III**) werden in 2 ml Methylenchlorid gelöst und mit 75 mg N-Ethyl-N'-3-(dimethylamino)propyl-carbodiimid und 50 mg 1H-1,2,3-benzotriazol-1-ol versetzt. Anschließend wird eine äquivalente Menge Dimethylamin (2 molare Lösung in absolutem THF) und 50 mg Triethylamin zugegeben. Nach 10 min bei RT wird an Kieselgel chromatographiert. Ausbeute: 80 mg (75%).
RF-Wert: 0,26 (a)

Analog Beispiel 1 wurden hergestellt:

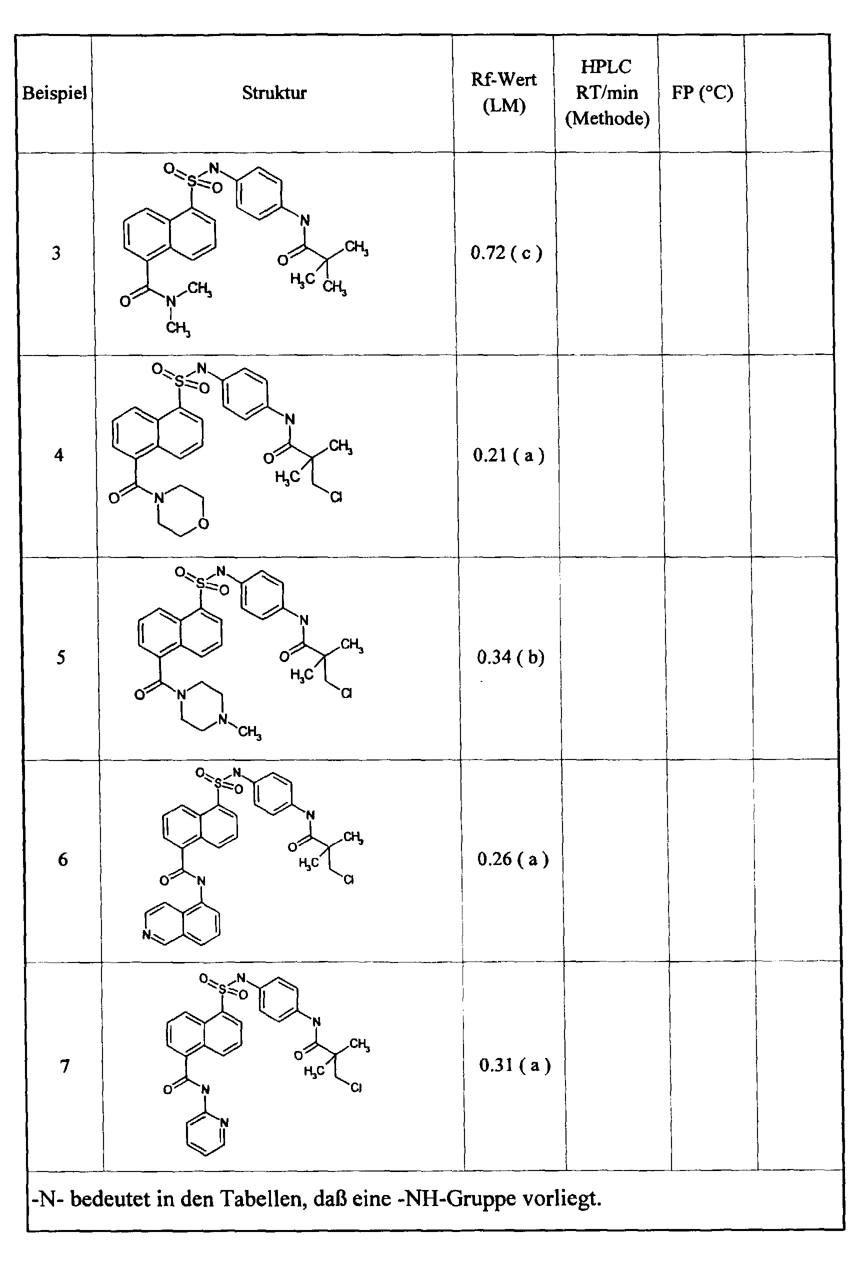

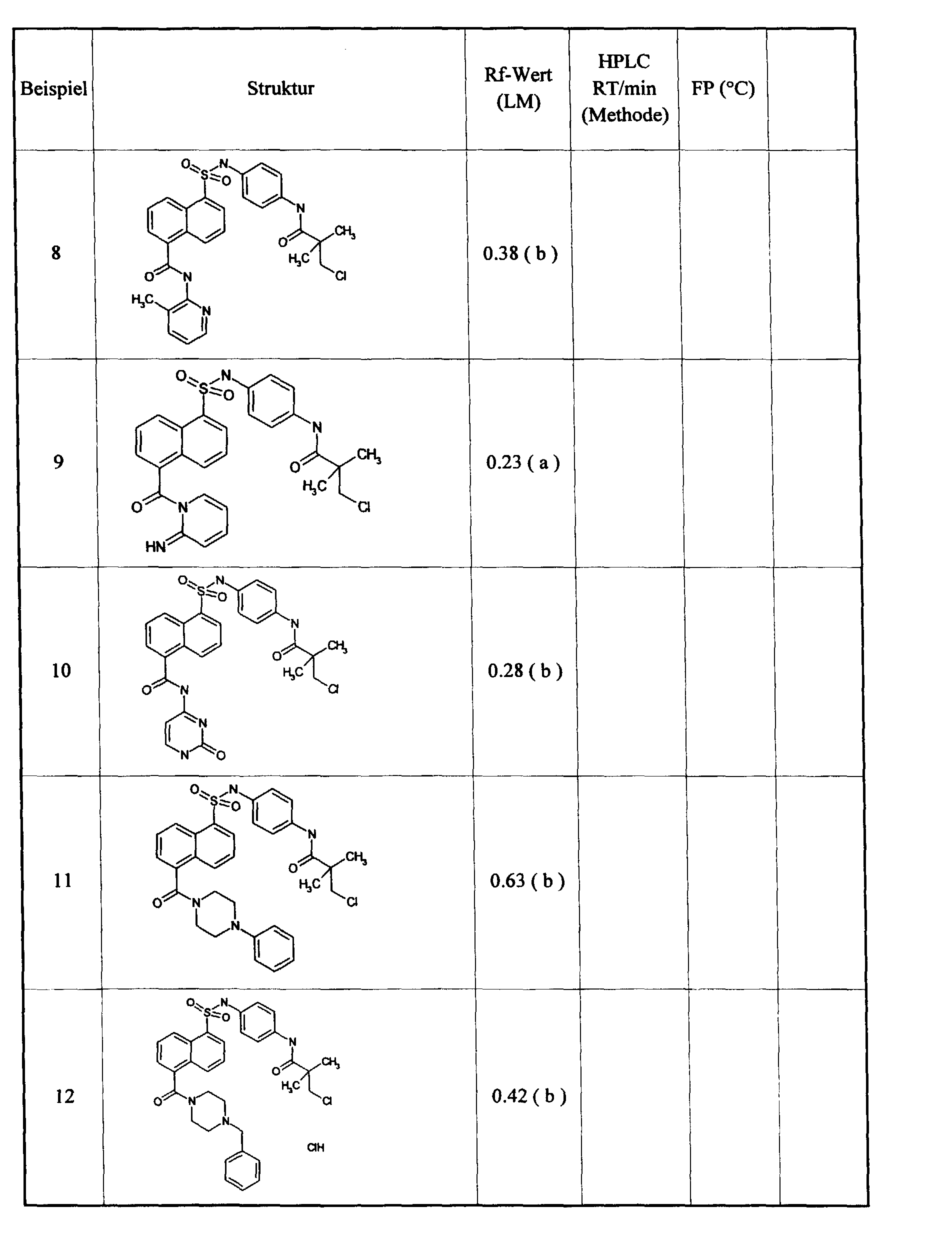

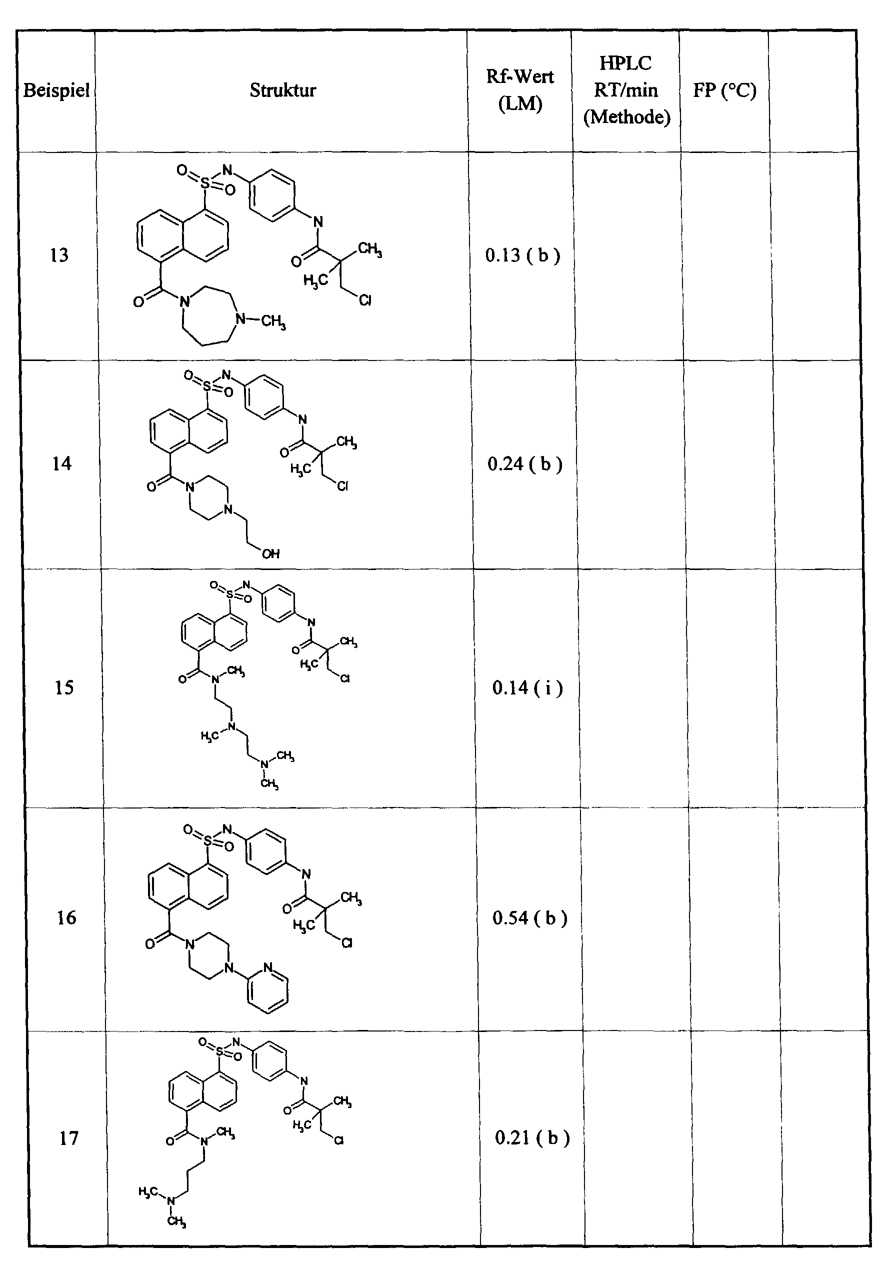

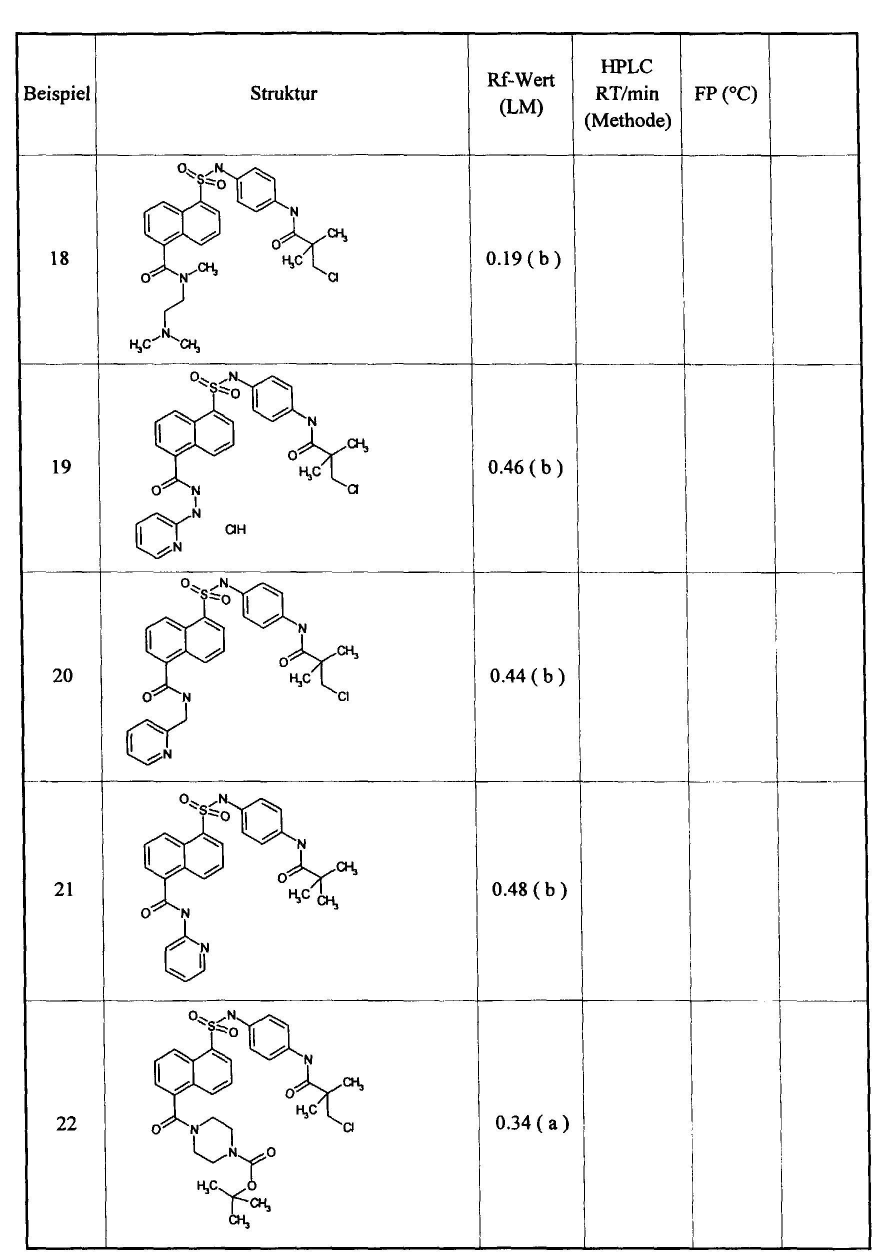

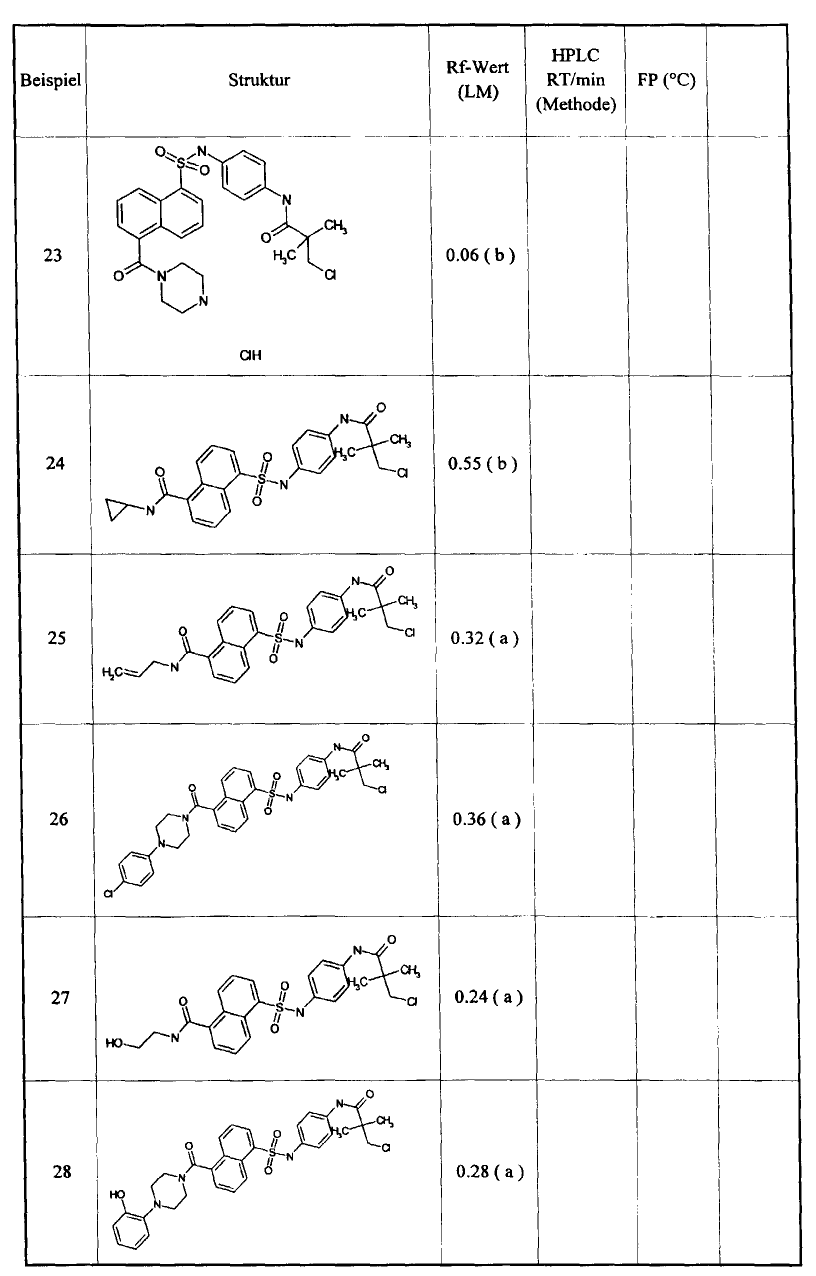

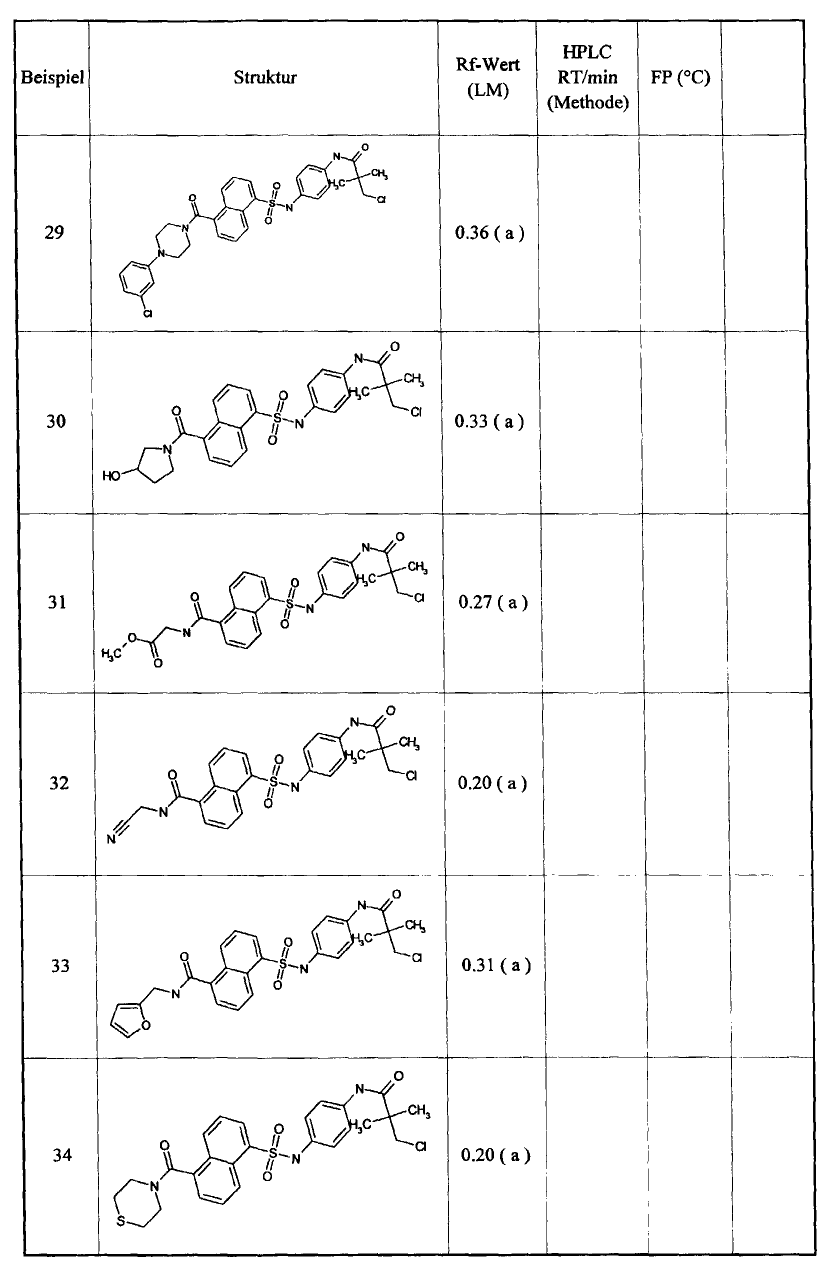

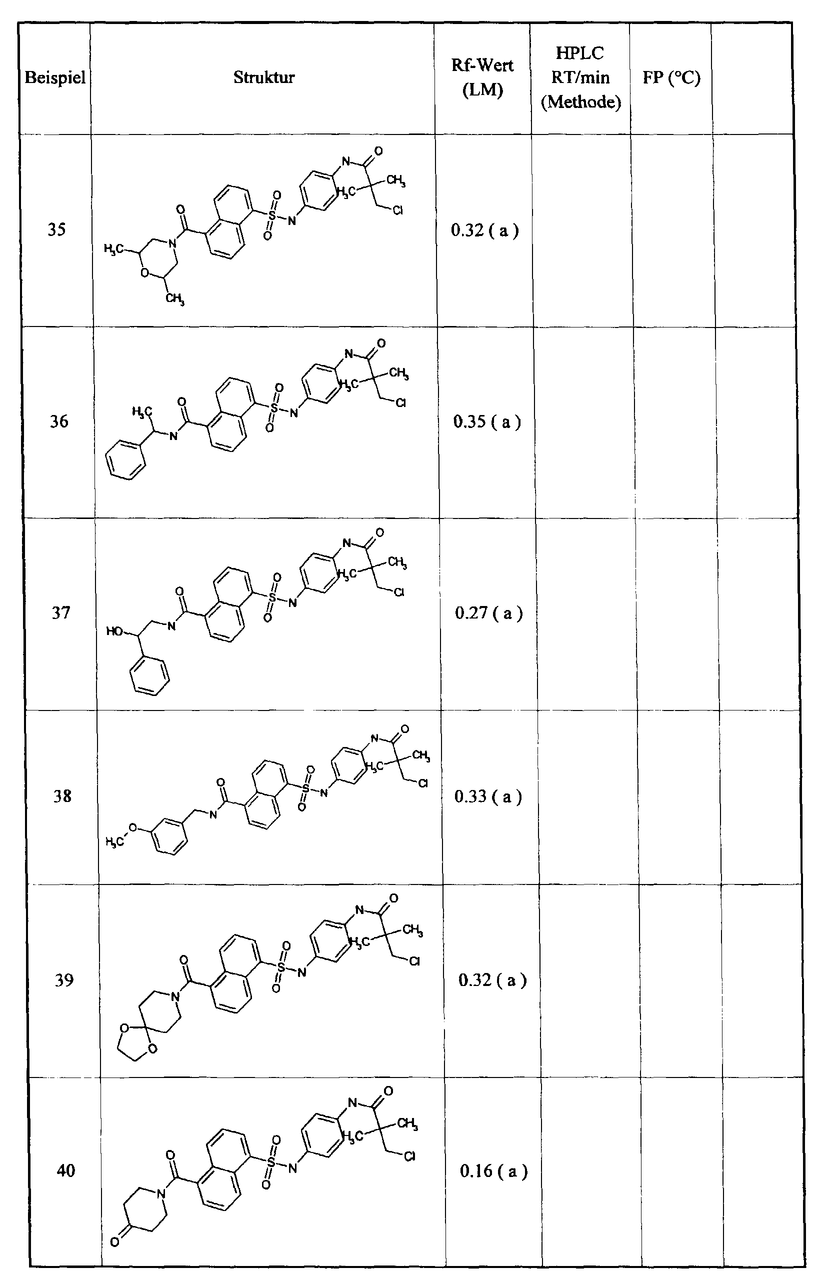

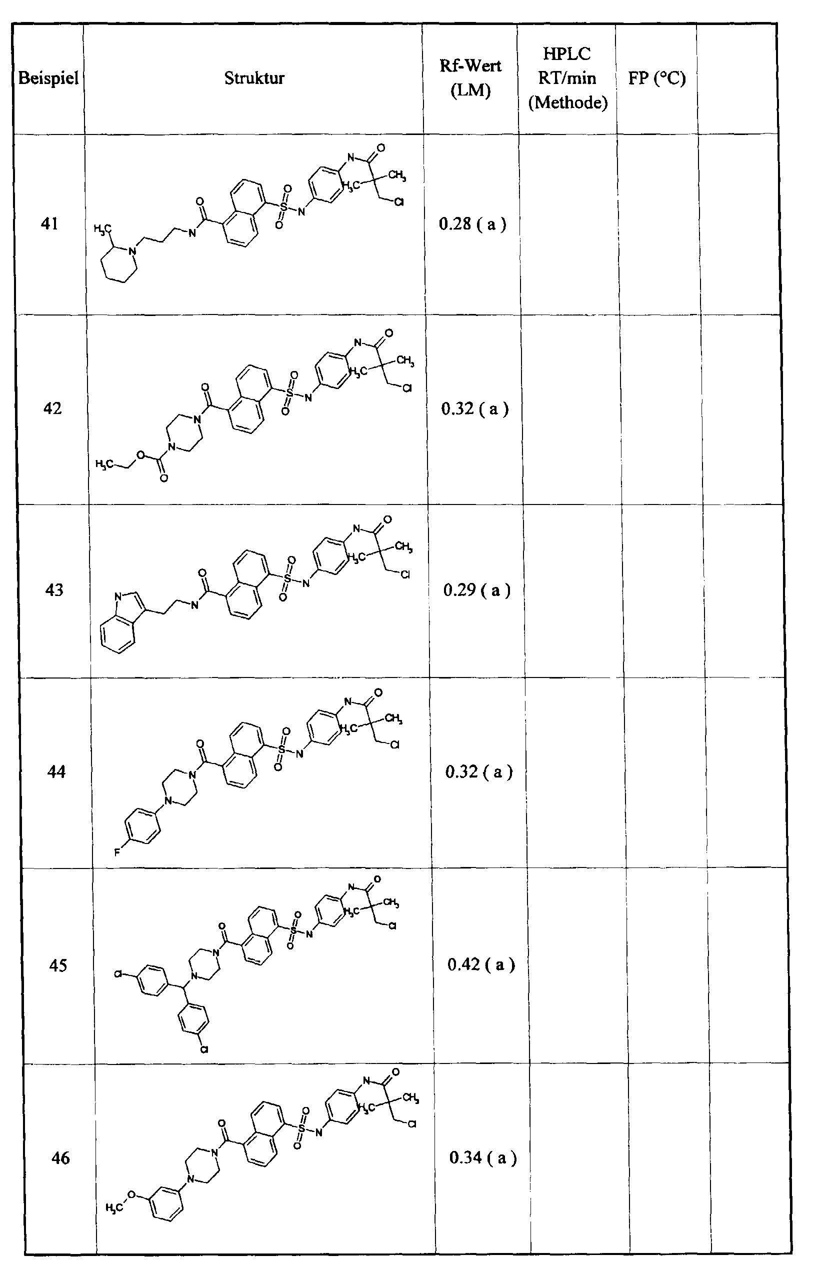

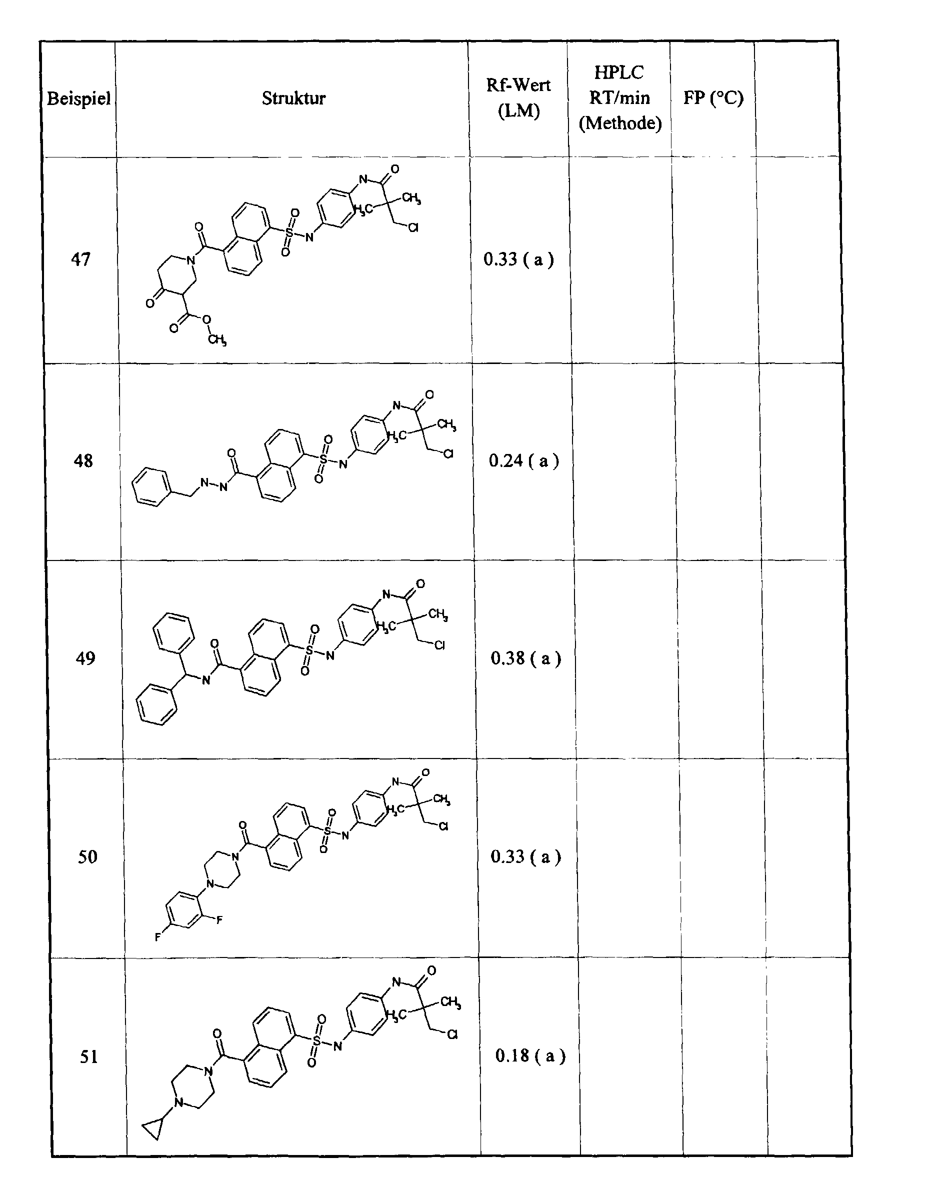

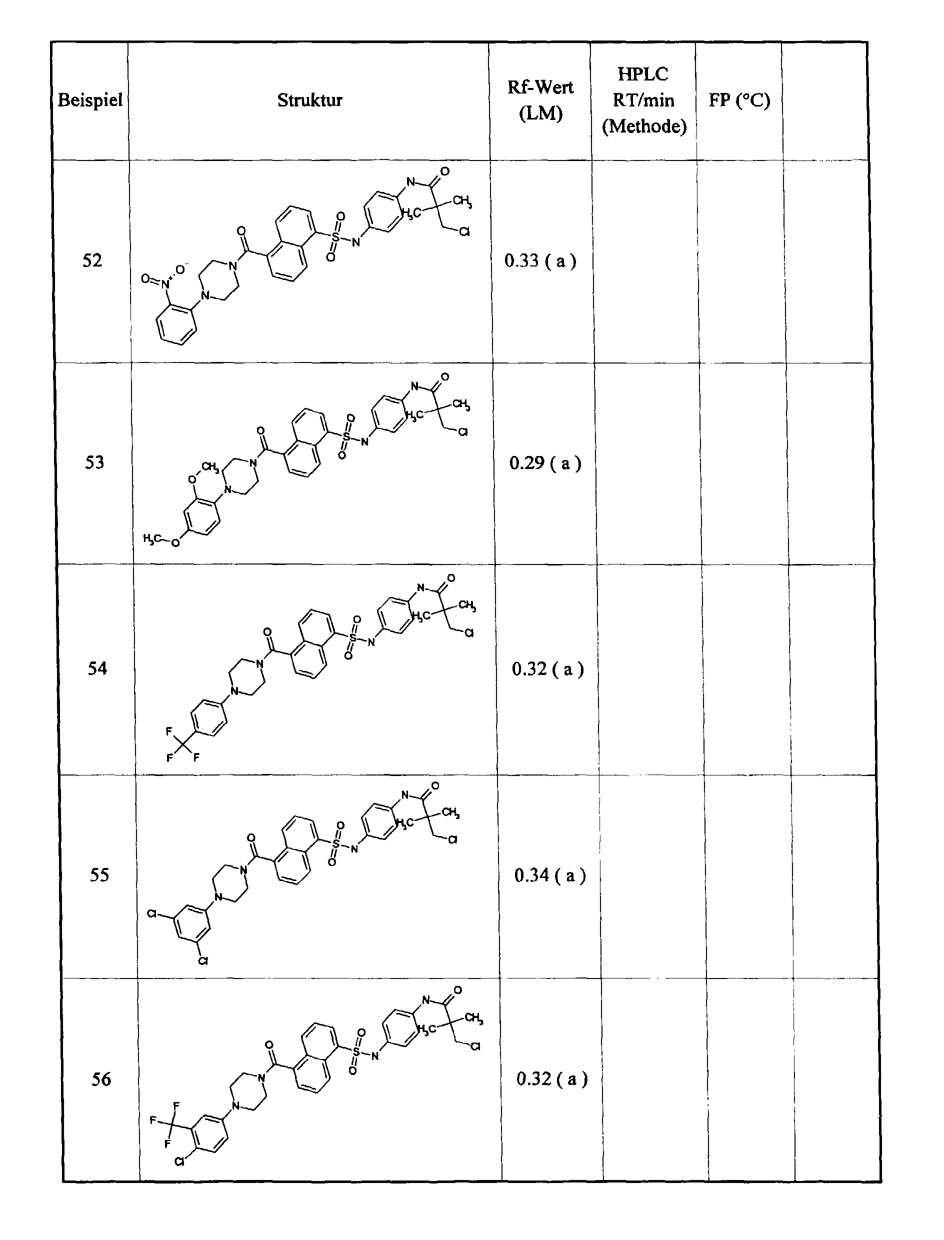

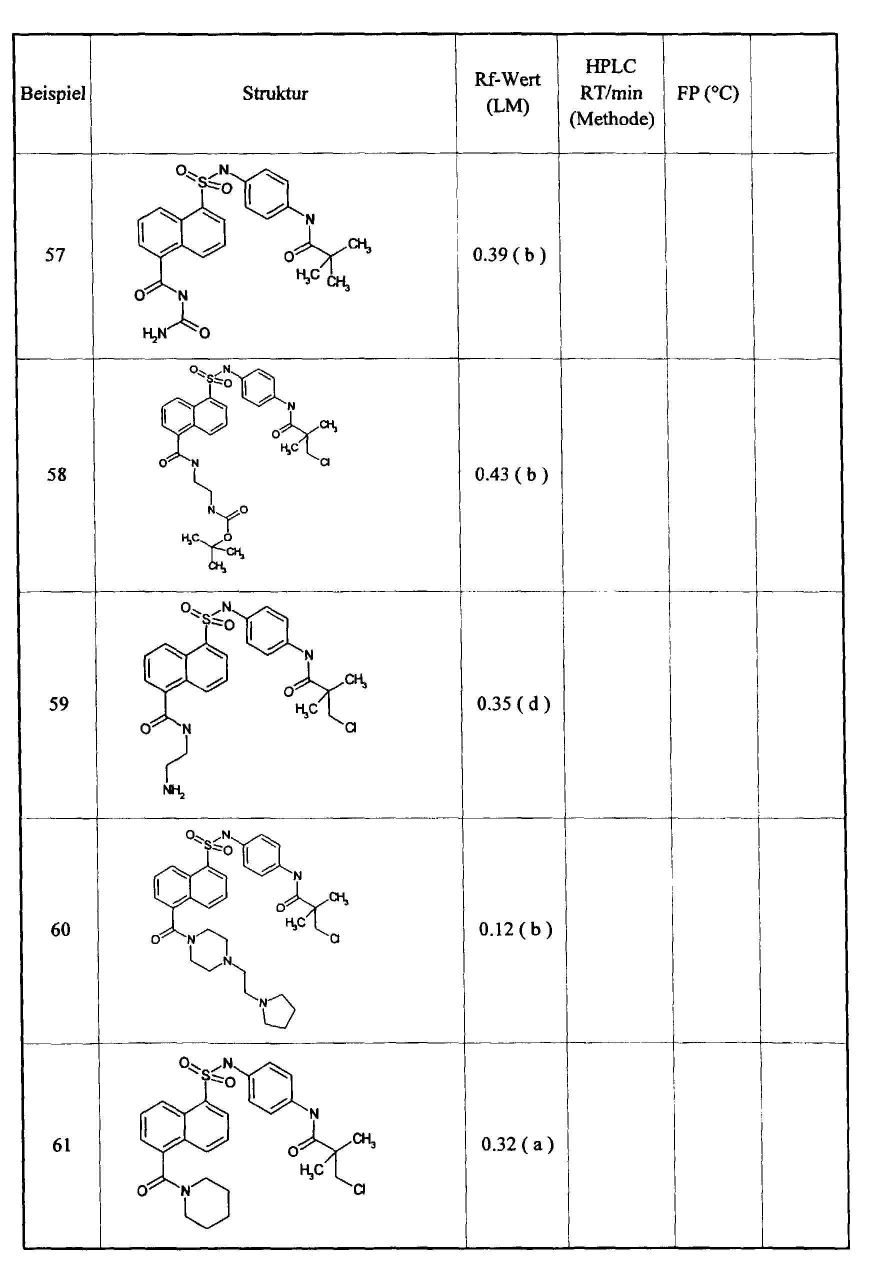

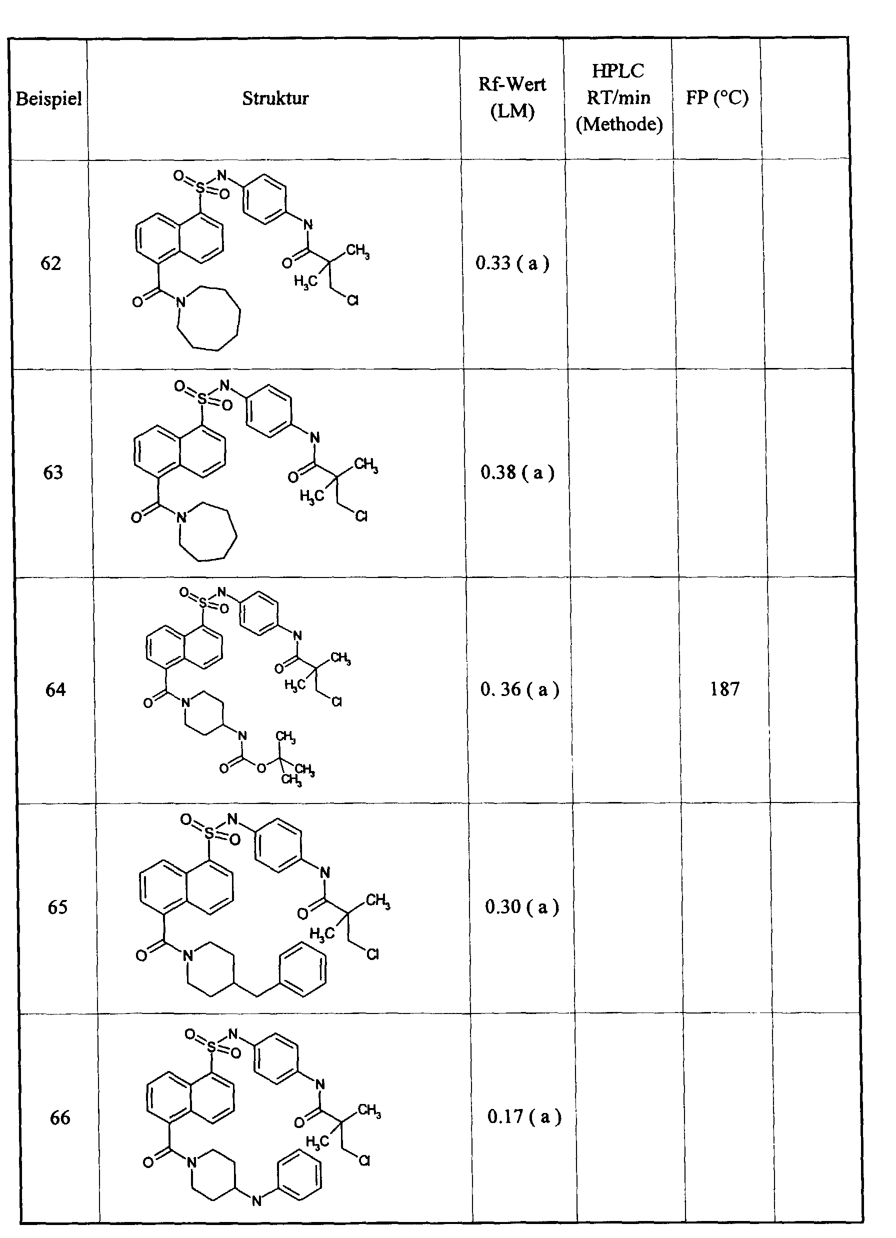

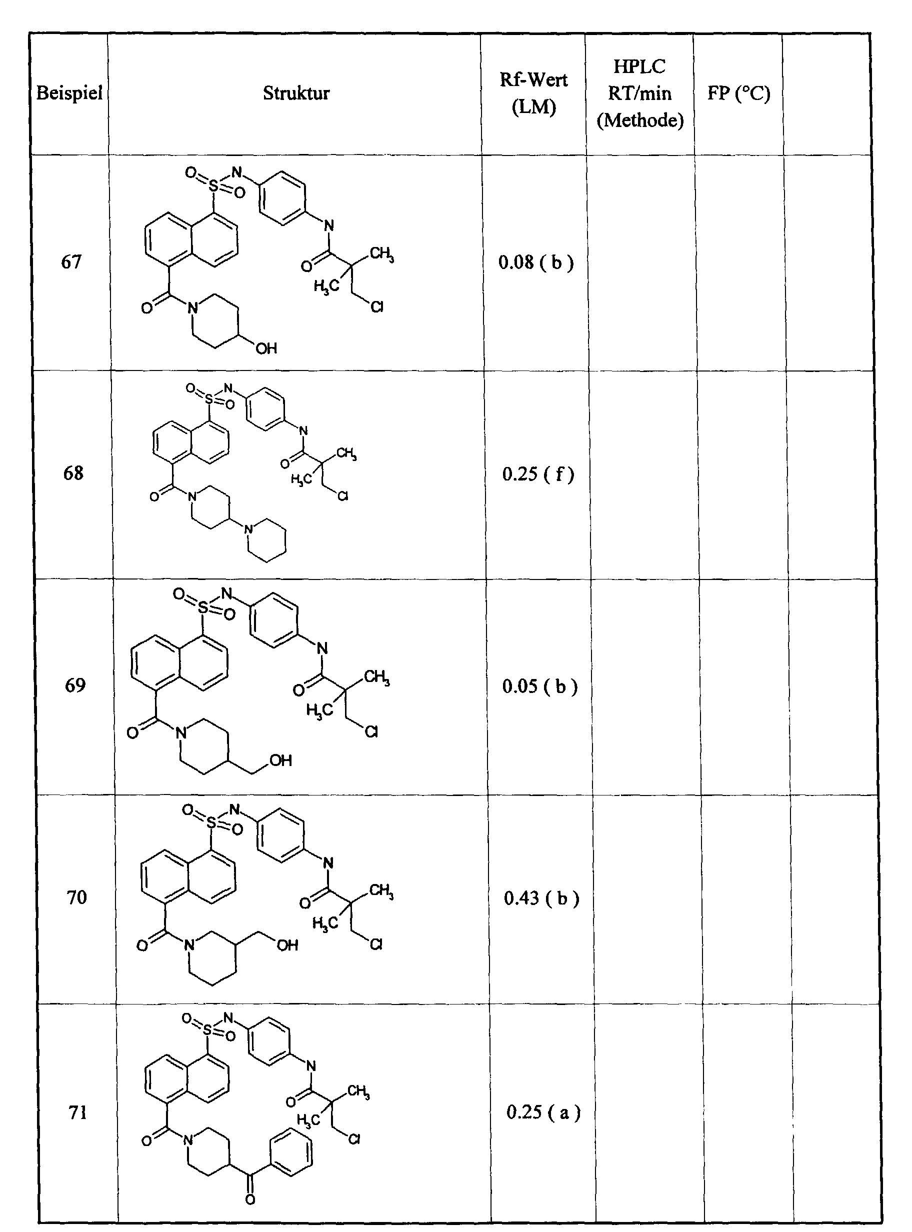

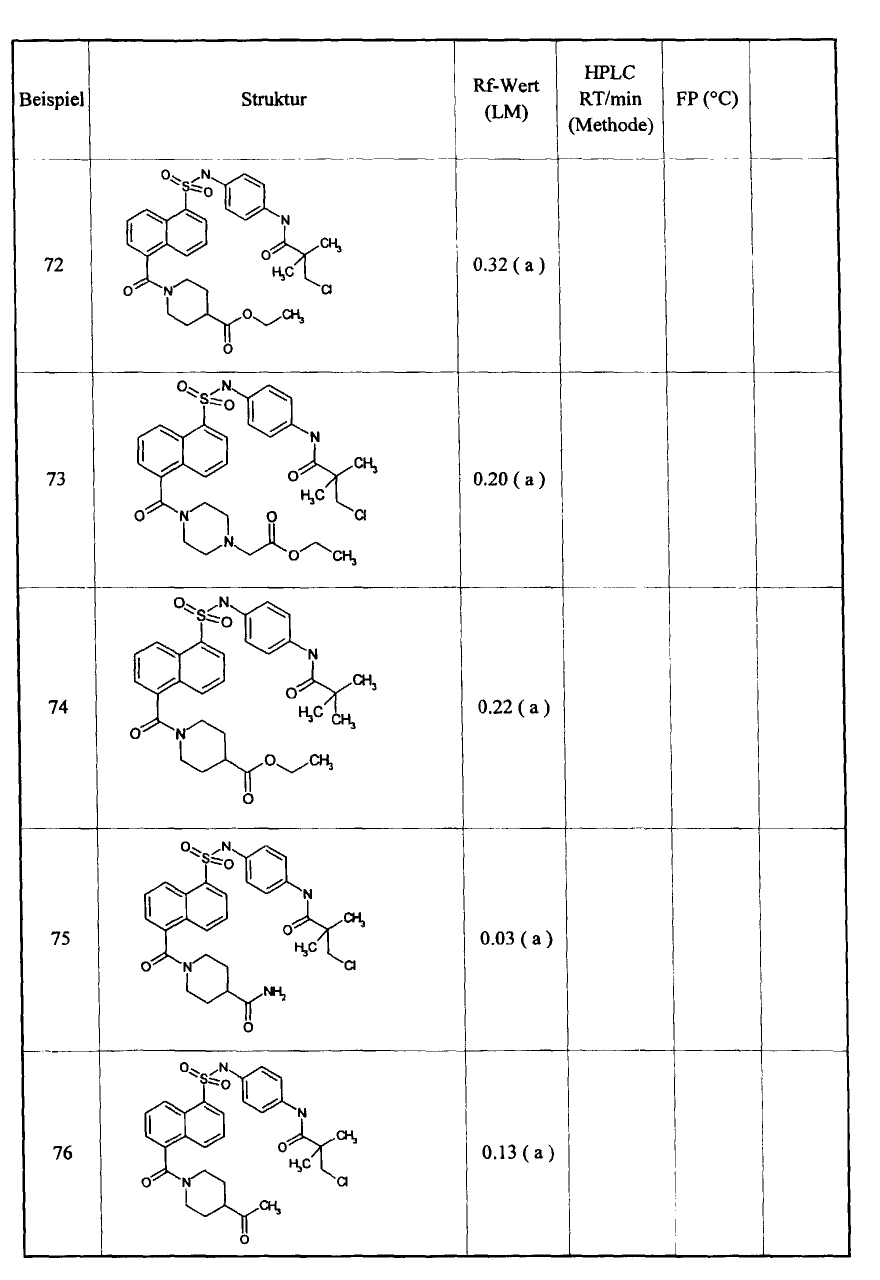

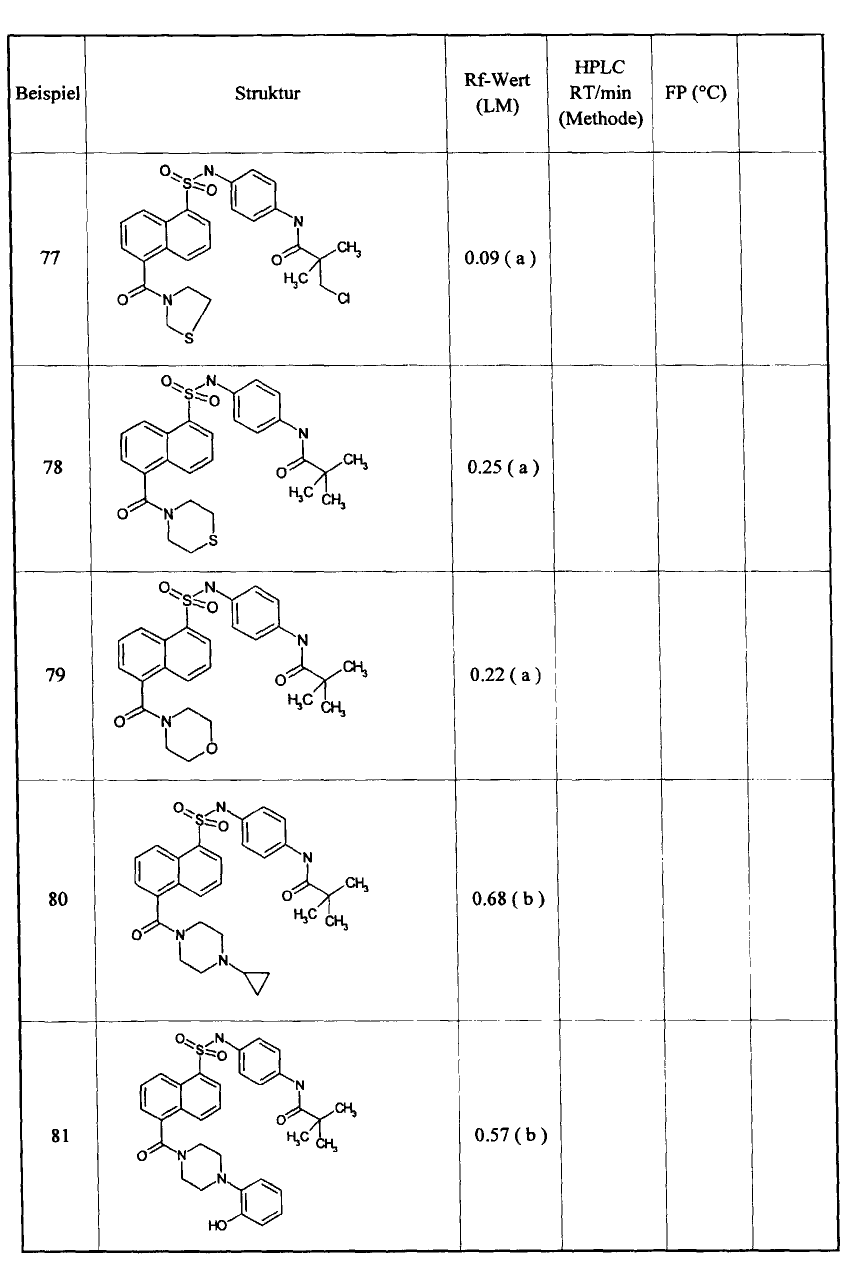

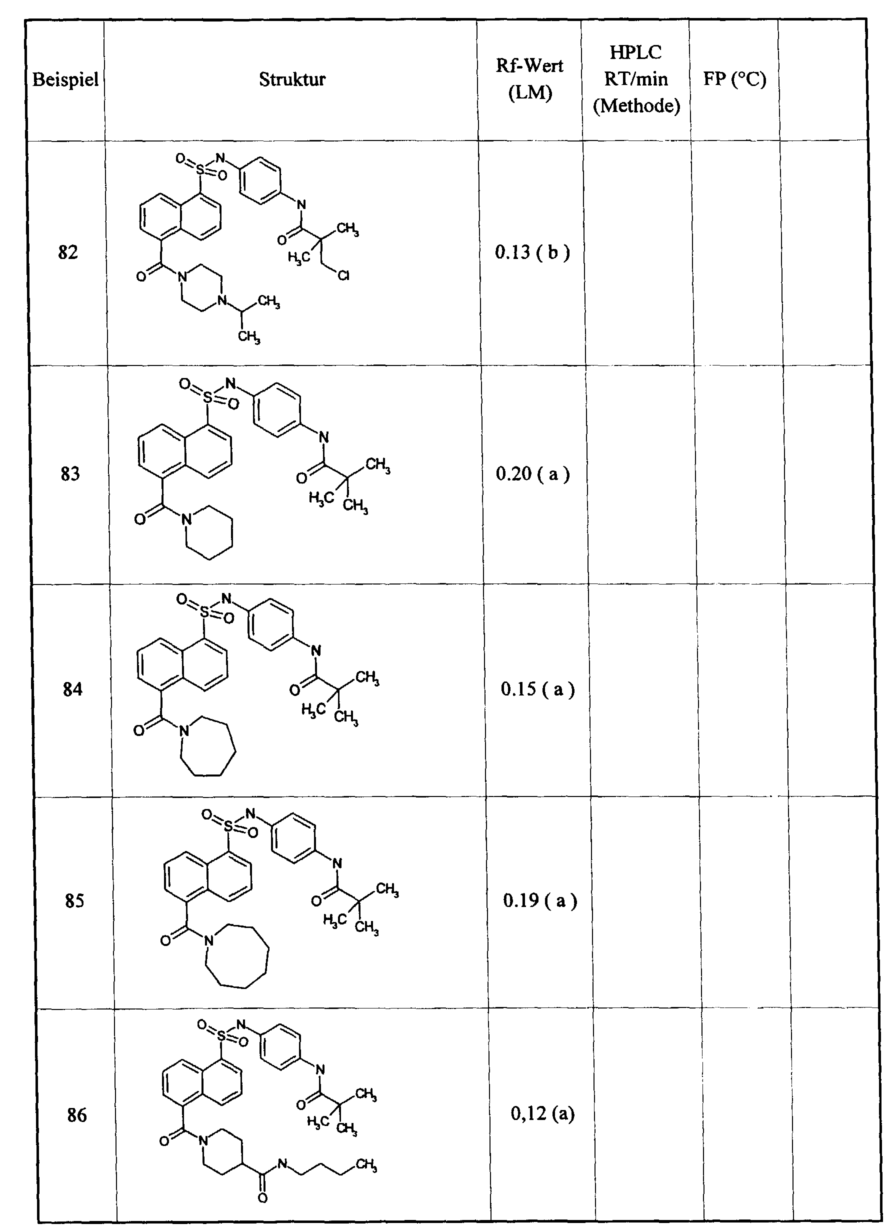

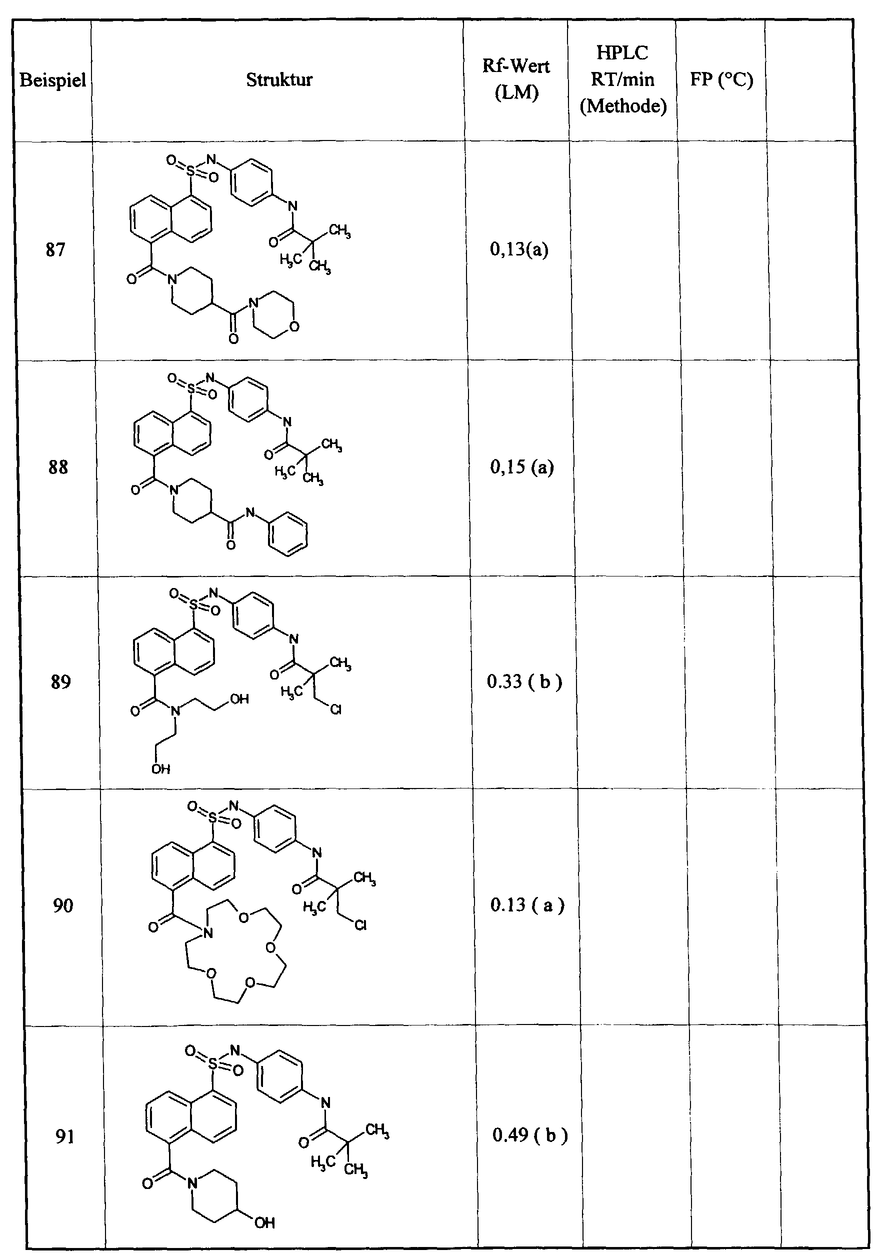

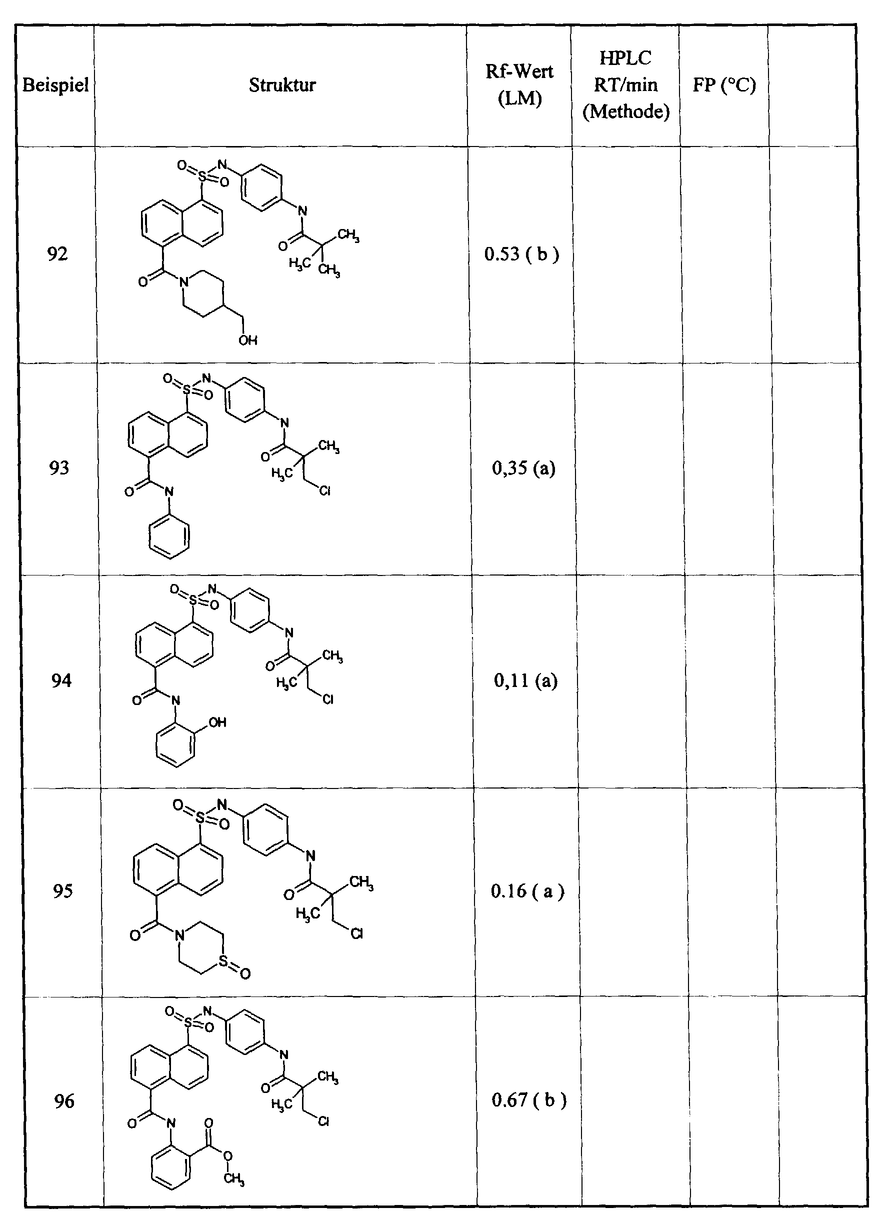

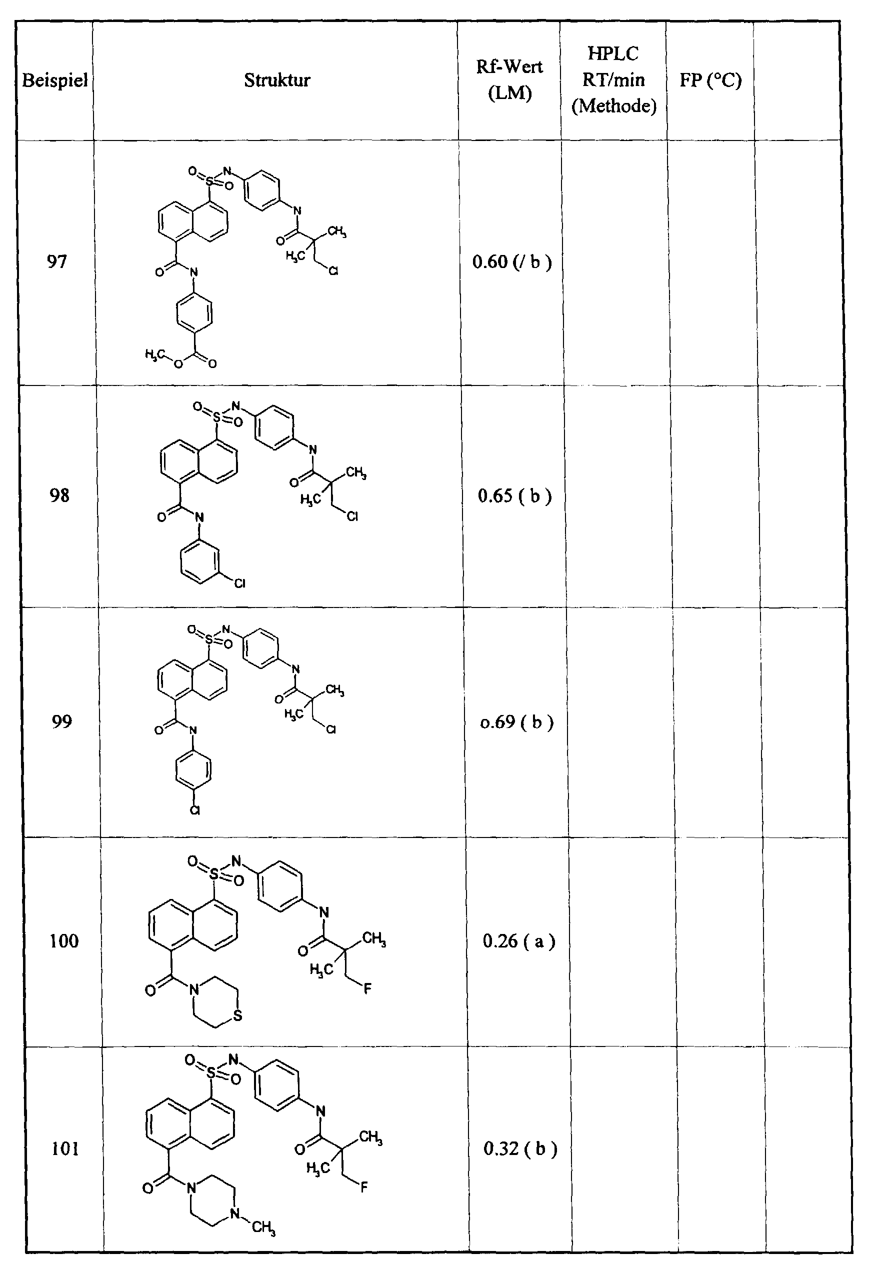

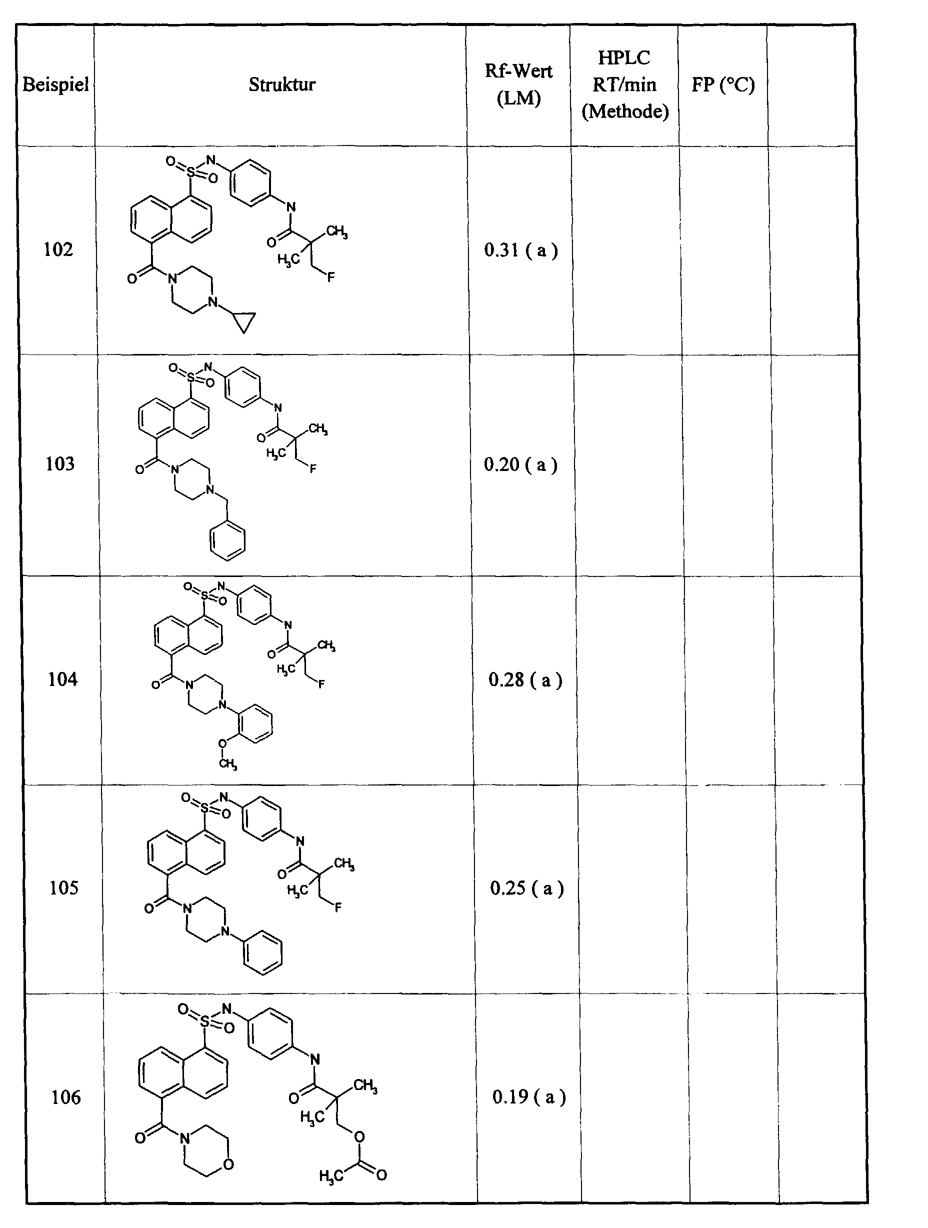

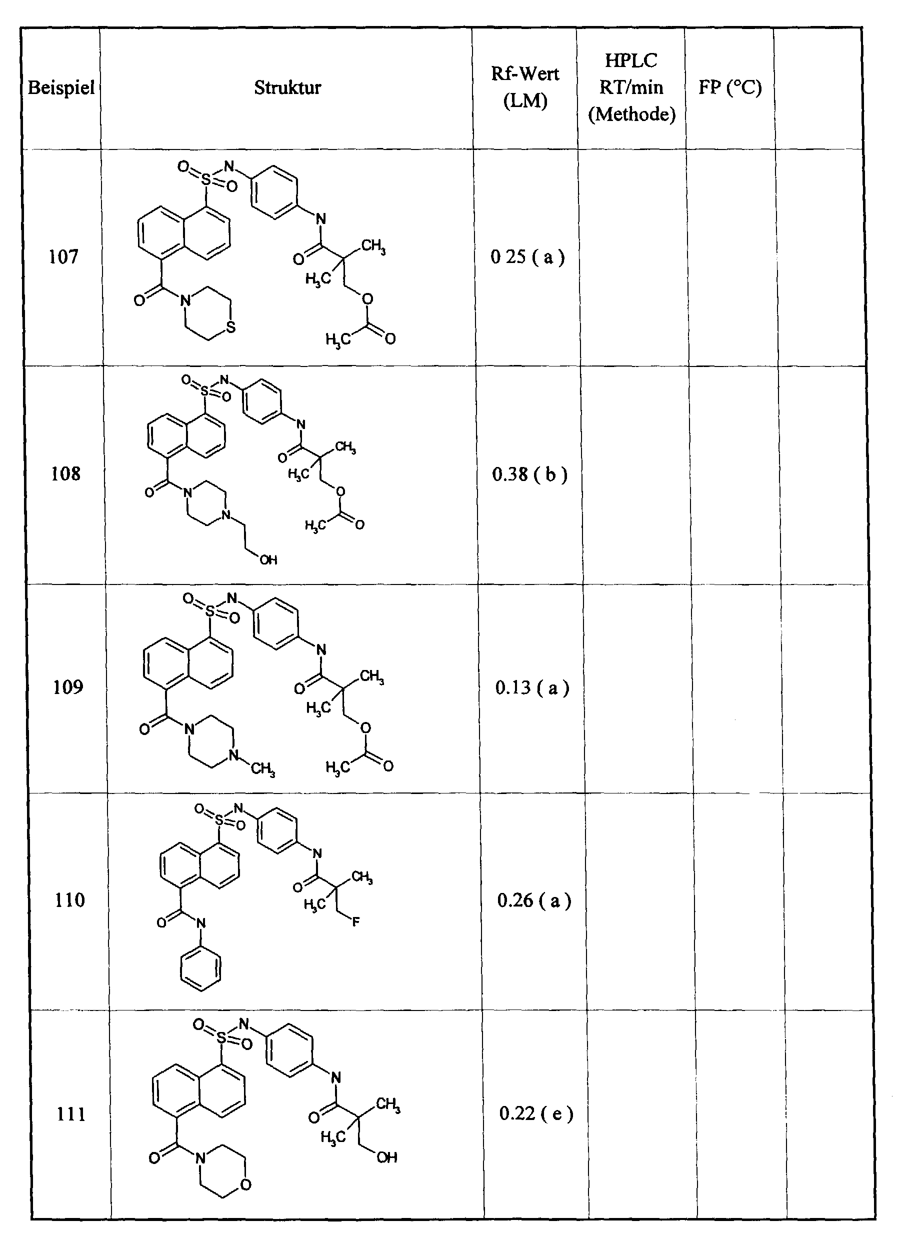

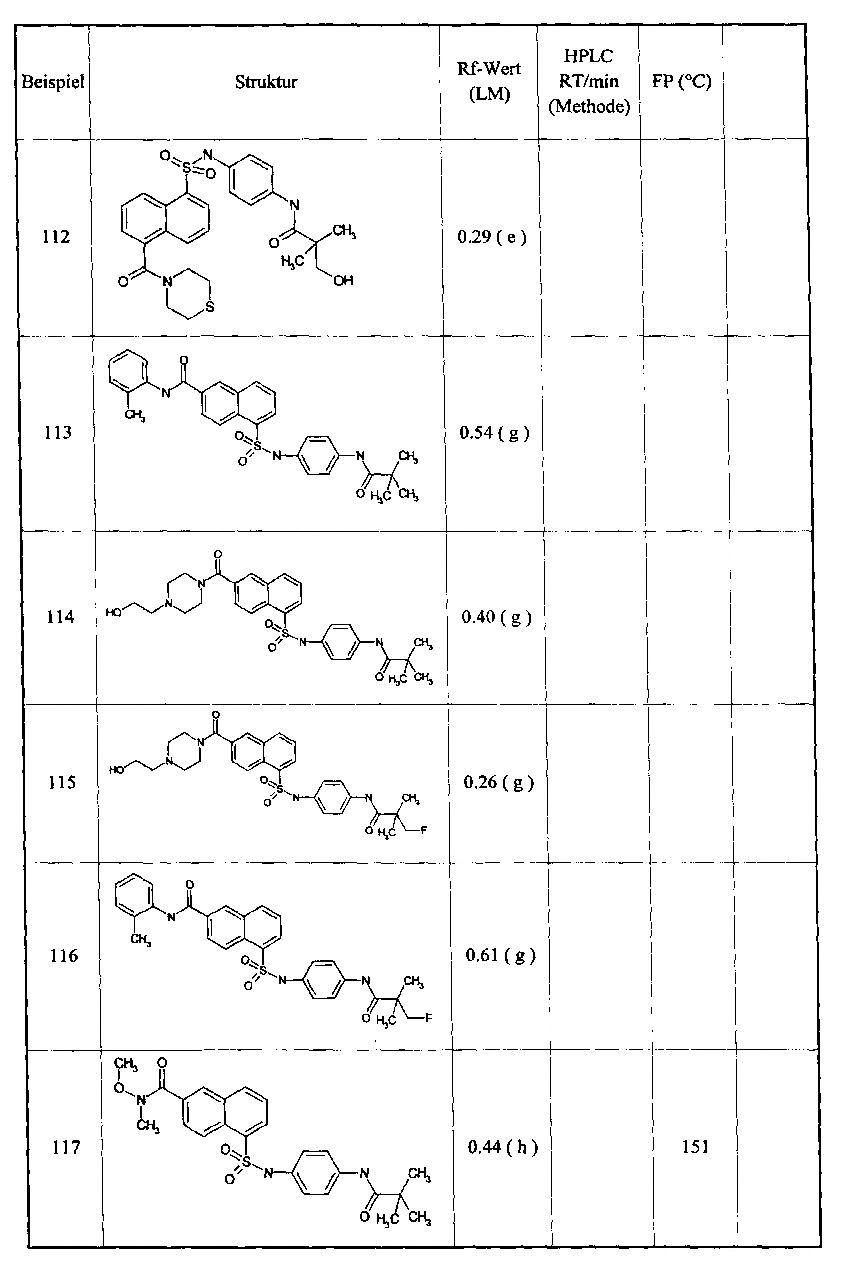

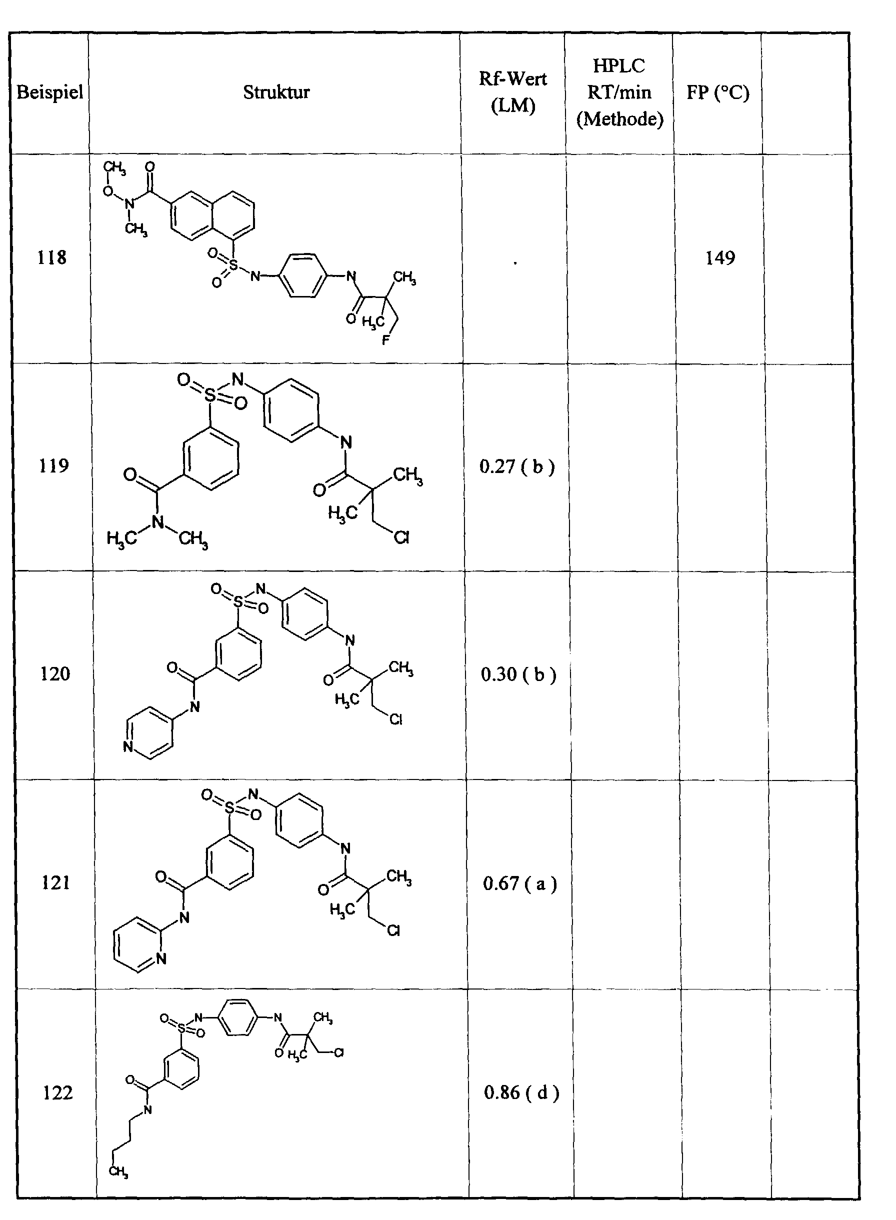

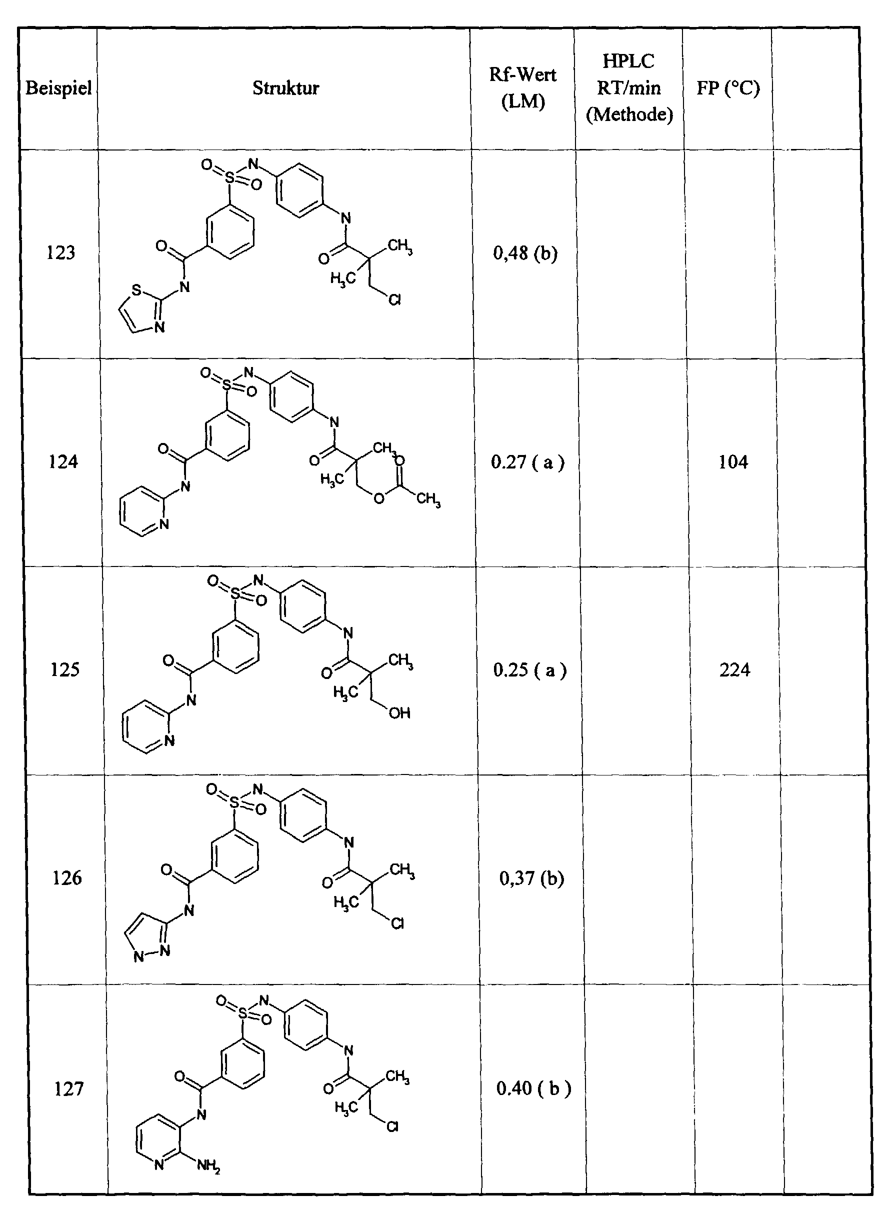

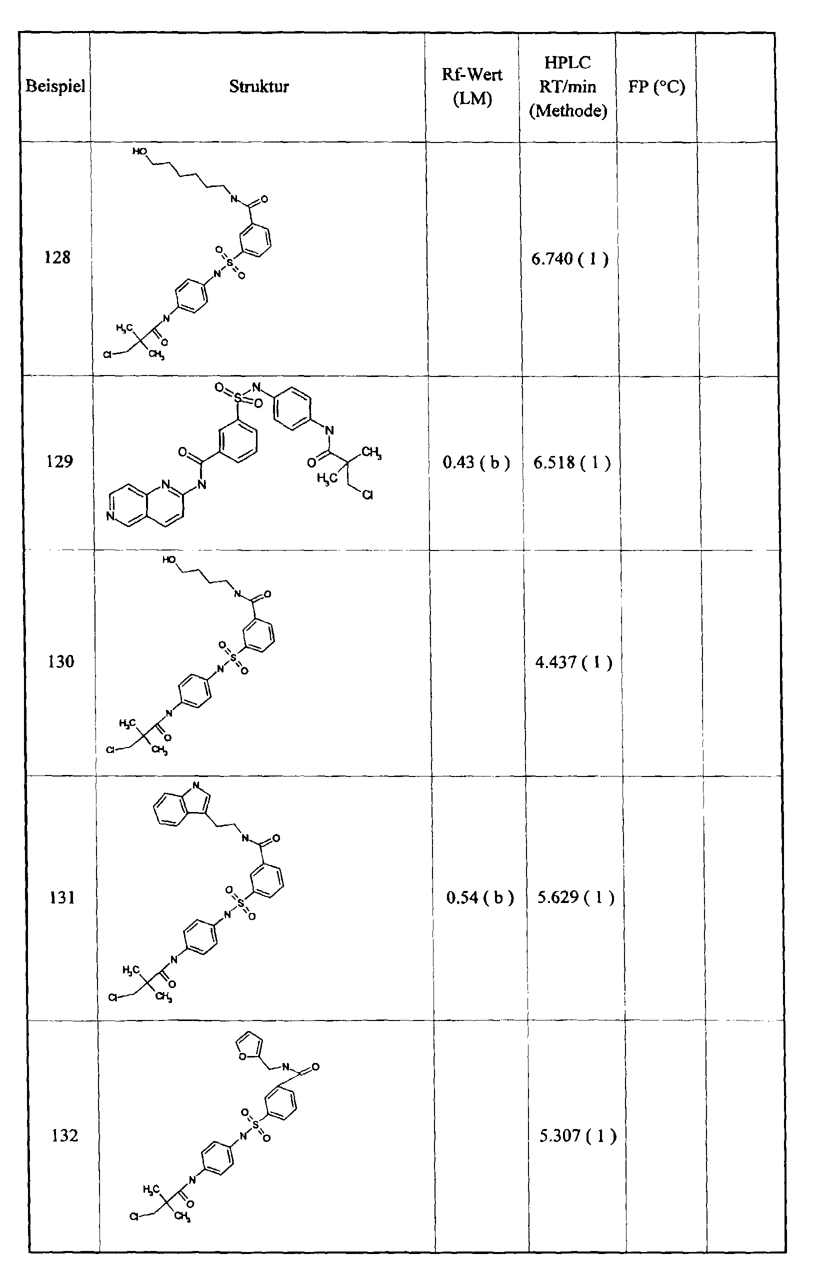

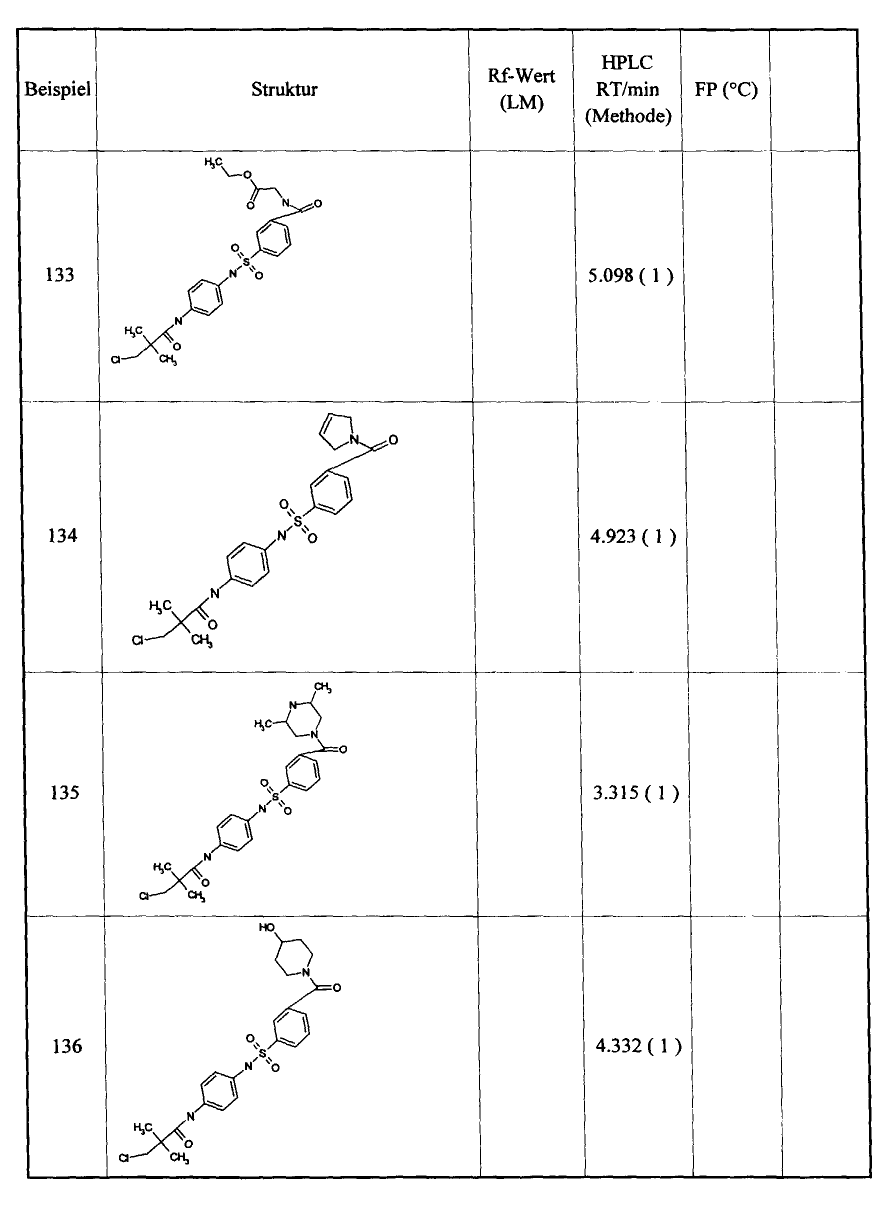

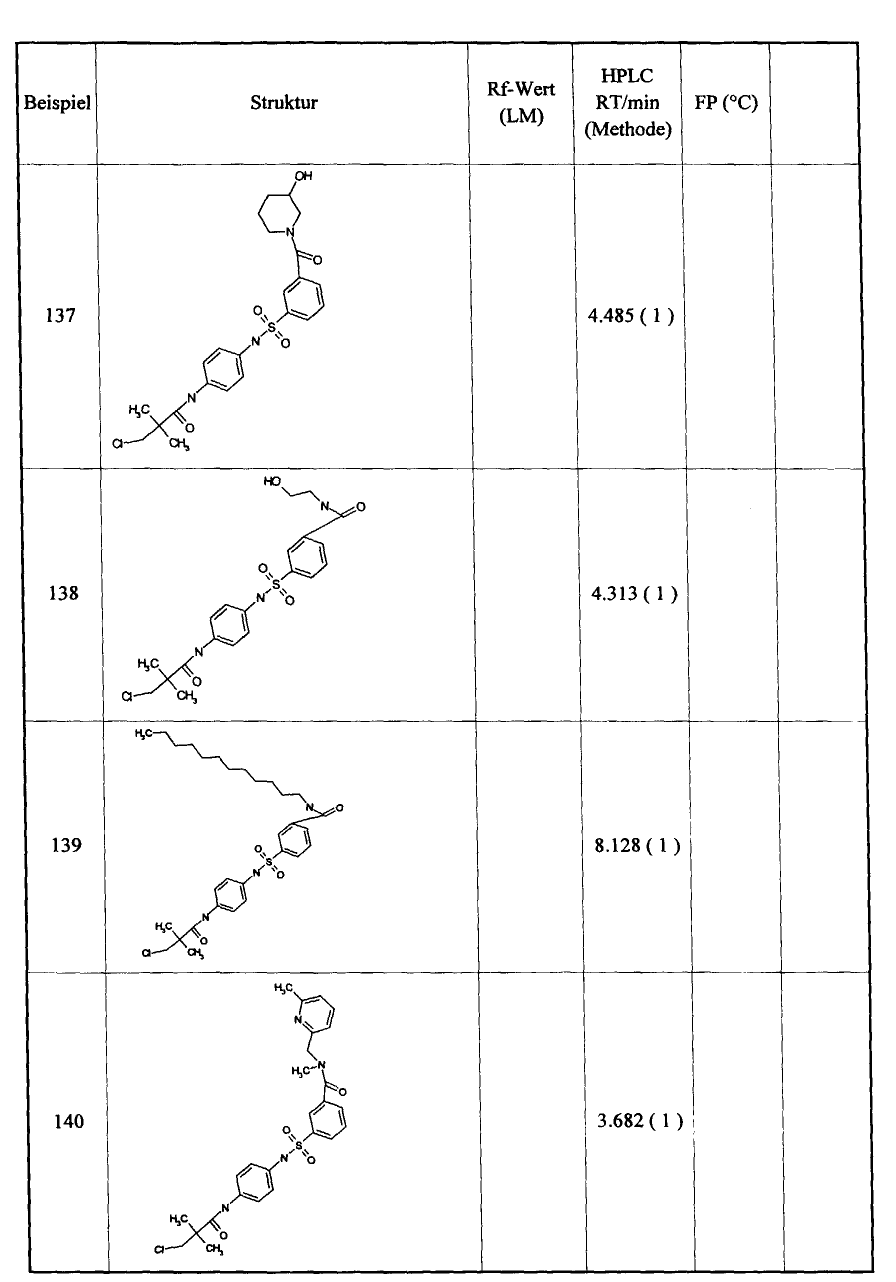

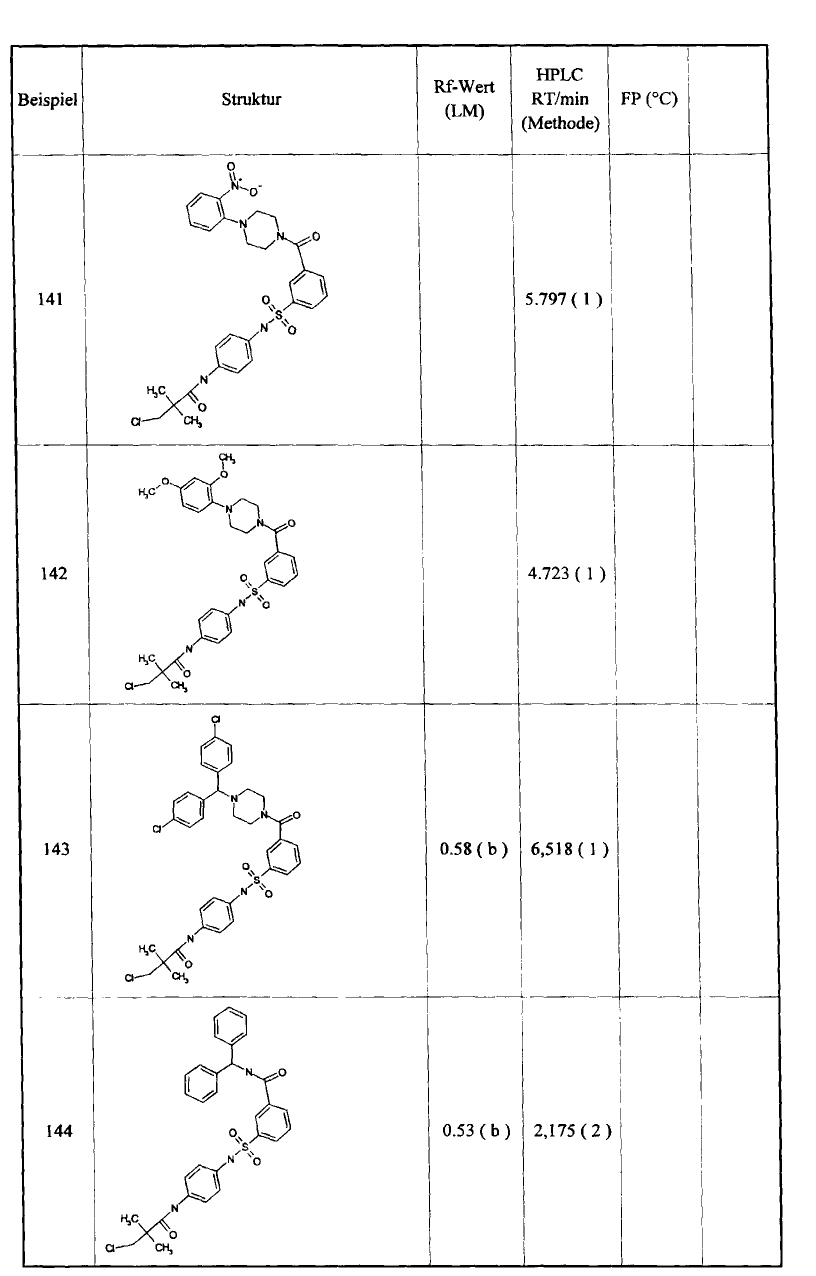

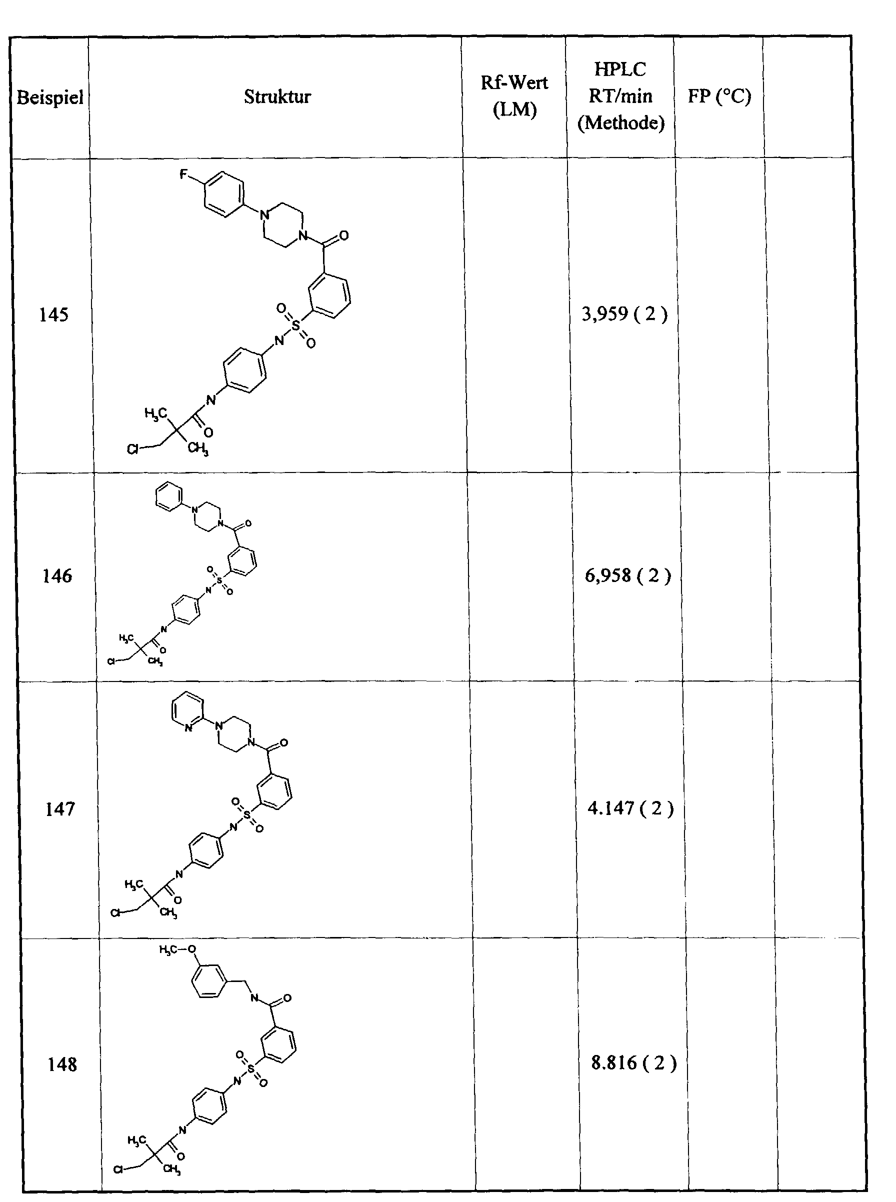

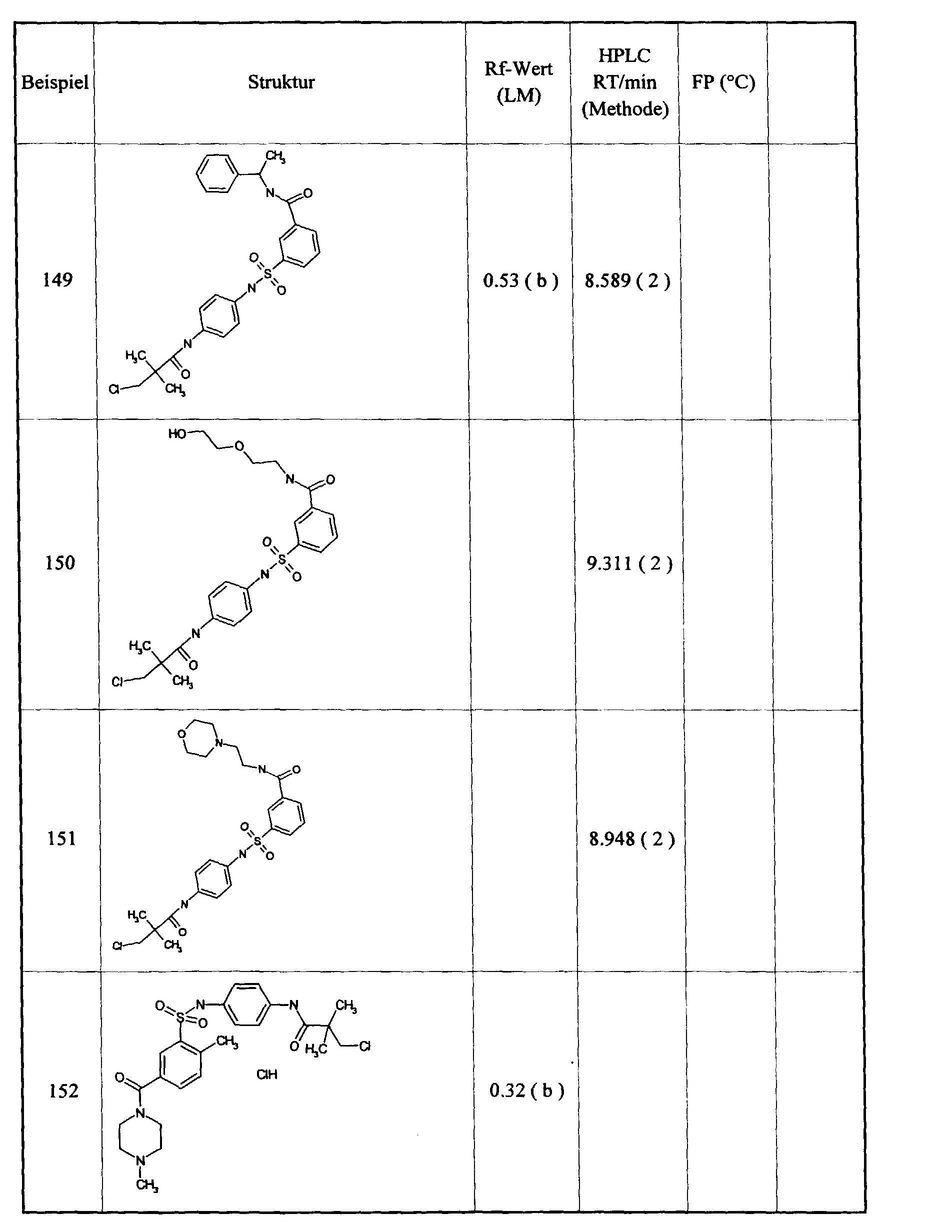

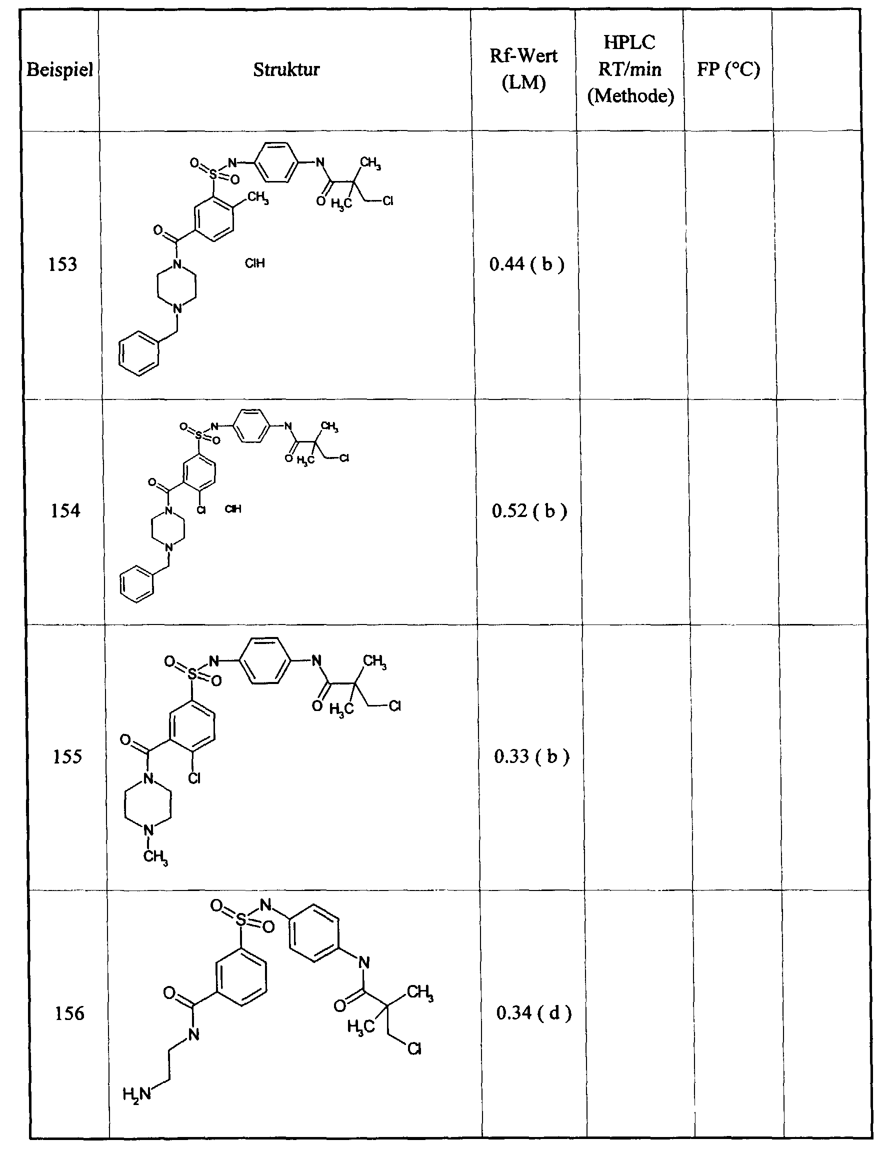

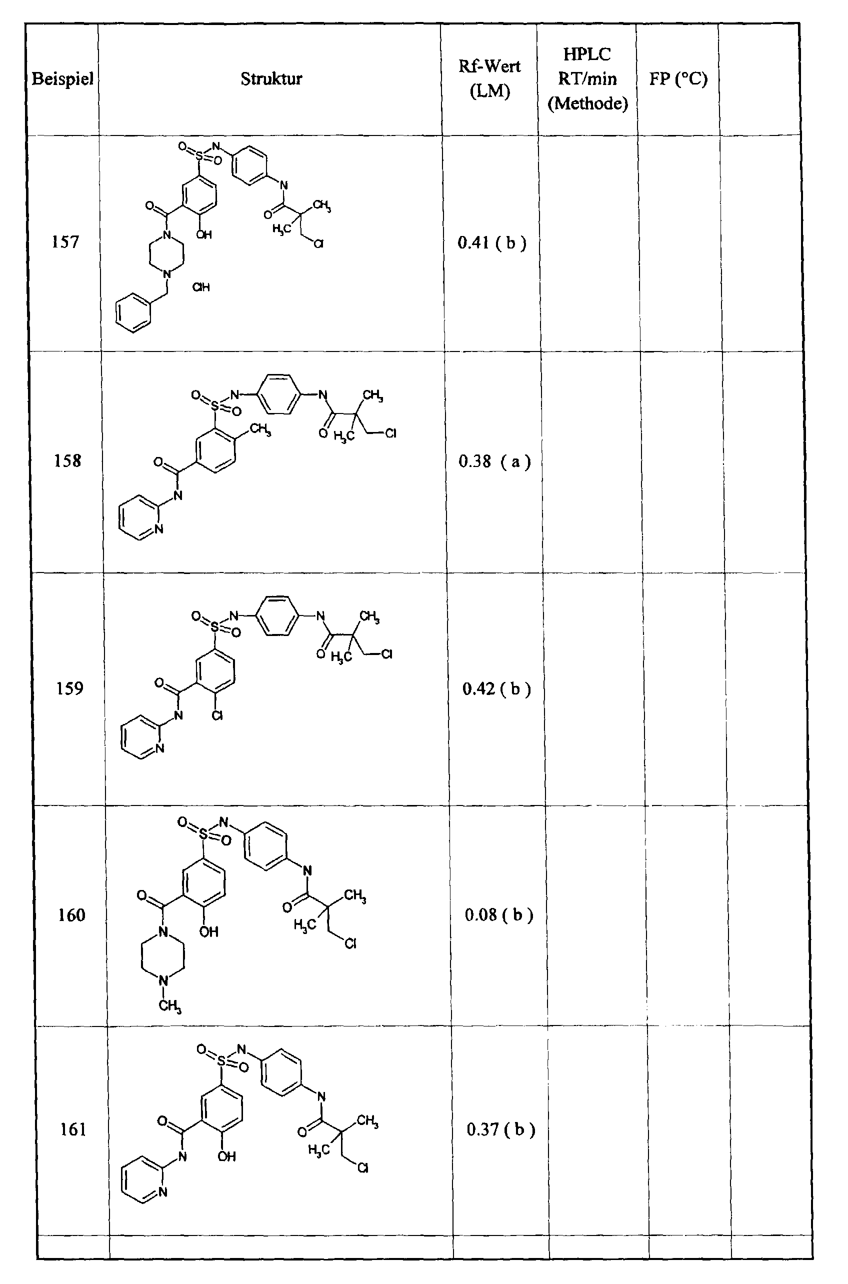

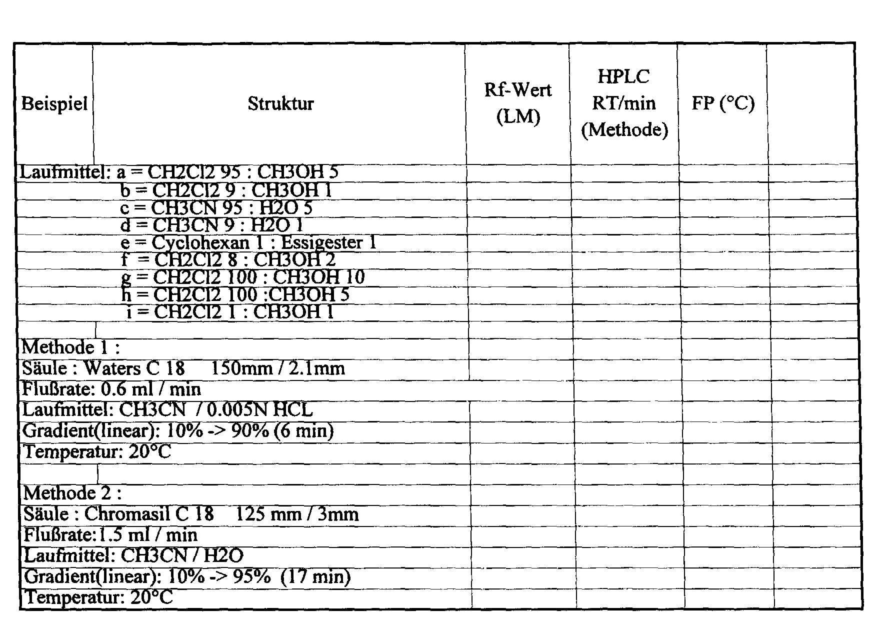

## Patentansprüche

1. Verbindungen der allgemeinen Formel ( I ): 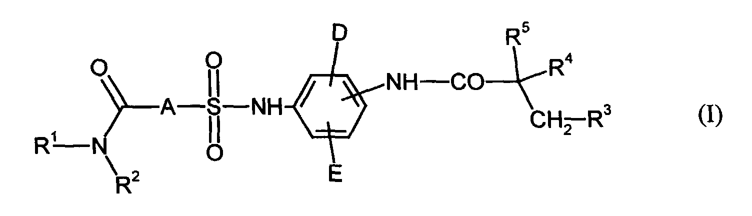 worin
R¹ für Wasserstoff oder eine geradkettige, verzweigte oder cyclische, gesättigte oder ungesättigte Kohlenwasserstoffgruppe mit 1 bis 14 Kohlenstoffatomen steht, worin gegebenfalls ein bis zwei Kohlenstoffatome in der Kette durch O oder S ersetzt sein können, und die durch ein bis drei Substituenten substituiert sein kann, die aus der Gruppe ausgewählt werden, die aus Hydroxy, Cyano, -N(R⁶)R⁷, (C₁-C₆)Alkoxycarbonyl, (C₆-C₁₀)Aryl und 5- bis 10-gliedrigem Heteroaryl besteht, wobei (C₆-C₁₀)Aryl und 5- bis 10-gliedriges Heteroaryl ihrerseits durch ein bis drei Substituenten substituiert sein können, die aus Hydroxy, (C₁-C₆)Alkyl, (C₁-C₆)Alkoxy, (C₁-C₆)-Alkoxycarbonyl, Halogen und Halogen(C₁-C₆)alkyl ausgewählt werden, und worin
R⁶ und R⁷ gleich oder verschieden sind und für Wasserstoff, (C₁-C₆)Alkoxycarbonyl oder eine geradkettige, verzweigte oder cyclische, gesättigte oder ungesättigte Kohlenwasserstoffgruppe mit 1 bis 10 Kohlenstoffatomen, die gegebenfalls mit Di(C₁-C₆)Alkylamino substituiert ist, stehen, oder gemeinsam mit dem Stickstoffatom, an das sie gebunden sind, einen 3- bis 8-gliedrigen heterocyclischen Ring bilden, der gegebenfalls durch 1 bis 3 (C₁-C₆)Alkylgruppen substituiert sein kann und/oder ein oder zwei weitere Heteroatome ausgewählt aus O, S oder N enthalten kann,
oder
R¹ für (C₆-C₁₀)Aryl oder 5- bis 10-gliedriges Heteroaryl steht, die gegebenenfalls durch ein bis drei Substituenten substituiert sein können, die aus der Gruppe ausgewählt werden, die besteht aus (C₁-C₆)Alkyl, Hydroxy, (C₁-C₆)Alkoxycarbonyl, Halogen und Amino, oder
R¹ für (5- bis 10-gliedriges Heteroaryl)amino oder (C₆-C₁₀)Aryl(C₁-C₆)alkylamino steht,
R² für (C₁-C₆)Alkoxy, (C₁-C₆)Alkoxycarbonyl, Aminocarbonyl steht oder eine der oben genannten Bedeutungen von R¹ aufweisen kann und von dieser Bedeutung gleich oder verschieden sein kann,
oder
R¹ und R² gemeinsam mit dem Stickstoffatom, an das sie gebunden sind einen gesättigten oder ungesättigten Ring bilden mit bis zu 14 C-Atomen, dessen Ringkohlenstoffatome gegebenfalls durch 1 bis 4 Reste ausgewählt aus der Gruppe, die aus O , S, S=O, C=O und N-R⁸ besteht, ersetzt sein können, und wobei der Ring gegebenfalls substituiert sein kann durch ein bis drei Substituenten, ausgewählt aus der Gruppe, die besteht aus Hydroxy, (C₁-C₆)-Alkyl, Benzyl, Hydroxy(C₁-C₆)alkyl, Phenylamino, Benzoyl, (C₁-C₆)Acyl, (C₁-C₆)Alkoxycarbonyl, Aminocarbonyl, Mono- oder Di(C₁-C₆)alkylaminocarbonyl, Morpholinocarbonyl, Phenylaminocarbonyl, (C₁-C₆)Alkoxycarbonyl(C₁-C₆)alkyl, (C₁-C₆)Alkoxyaminocarbonyl, (C₁-C₆)-Alkoxycarbonylamino, 1,3-Dioxolan-2,2-diyl, Piperidyl, Piperidino, Imino, worin
R⁸ für Wasserstoff, eine geradkettige, verzweigte oder cyclische, gesättigte oder ungesättigte Kohlenwasserstoffgruppe mit bis zu 10 Kohlenstoffatomen steht, die gegebenfalls durch 1 bis 3 Reste ausgewählt aus der Gruppe, die aus Hydroxy, Pyrrolidinyl, Pyridyl, Phenyl, Halogenphenyl, Halogen(C₁-C₃)alkylphenyl und (C₁-C₆)-Alkoxycarbonyl besteht, substituiert sein kann, oder
R⁸ für (C₁-C₆)Alkoxycarbonyl, (C₆-C₁₀)Aryl oder 5- bis 10-gliedriges Heteroaryl steht, wobei (C₆-C₁₀)Aryl und 5- bis 10-gliedriges Heteroaryl ihrerseits durch ein bis drei Substituenten substituiert sein können, die aus der Gruppe bestehend aus Halogen, Hydroxy, (C₁-C₆)Alkoxy, Nitro oder Fluor(C₁-C₃)alkyl ausgewählt werden,
R³ für Wasserstoff, Halogen, Hydroxy, (C₁-C₆)Acyl, Benzoyl oder (C₁-C₆)Acyloxy steht,
R⁴ und R⁵ unabhängig voneinander eine (C₁-C₃)Alkylgruppe sind oder gemeinsam mit dem Kohlenstoffatom, an das sie gebunden sind einen 3-bis 6-gliedrigen carbocyclischen Ring bilden,
A für einen zweiwertigen (C₆-C₁₀)Arylrest oder einen zweiwertigen 5-bis 10-gliedrigen Heteroarylrest steht die gegebenenfalls substituiert sein können durch ein bis drei Substituenten, die aus der Gruppe ausgewählt werden, die aus (C₁-C₃)Alkyl, Hydroxy oder Halogenatomen besteht, und
D und E gleich oder verschieden sind und für Wasserstoff, Fluor, Chlor, Nitro, Cyano, Hydroxy oder für (C₁-C₃)Alkyl, (C₁-C₃)Alkoxy oder (C₁-C₃)-Alkoxycarbonyl stehen,
sowie deren pharmazeutische verträgliche Salze.

2. Verbindungen der allgemeinen Formel (I) nach Anspruch 1, worin
R¹ für Wasserstoff, eine (C₃-C₆)-Cycloalkylgruppe, eine (C₂-C₆)-Alkenylgruppe oder eine geradkettige oder verzweigtkettige (C₁-C₁₂-Alkylgruppe steht, in der gegebenenfalls ein Kettenkohlenstoffatom durch Sauerstoff ersetzt ist, und die gegebenenfalls durch 1 bis 3 Substituenten substituiert sein kann, die aus der Gruppe ausgewählt werden, die aus Hydroxy, Cyano, -N-(R⁶)R⁷, (C₁-C₆)-Alkoxycarbonyl, (C₆-C₁₀)-Aryl und 5- bis 10-gliedrigem Heteroaryl besteht, wobei (C₆-C₁₀)-Aryl und 5- bis 10-gliedriges Heteroaryl ihrerseits durch 1 bis 3 Substituenten substituiert sein können, die ausgewählt werden aus (C₁-C₃)-Alkoxy und (C₁-C₃)-Alkyl, und worin
R⁶ und R⁷ gleich oder verschieden sind und für Wasserstoff, (C₁-C₆)-Alkoxycarbonyl und/oder (C₁-C₆)Alkyl, das gegebenenfalls durch Di(C₁-C₆)Alkylamino substituiert ist, stehen, oder gemeinsam mit dem Stickstoffatom, an das sie gebunden sind, einen 3- bis 6-gliedrigen heterocyclischen Ring bilden, der gegebenenfalls ein zusätzliches Sauerstoffatom enthalten kann,
oder
R¹ für Phenyl oder 5- bis 10-gliedriges Heteroaryl, das 1 bis 2 Heteroatome ausgewählt aus Stickstoff und Schwefel enthalten kann, steht, die jeweils gegebenenfalls durch 1 bis 2 Substituenten, ausgewählt aus Amino, (C₁-C₃)-Alkyl, Hydroxy, (C₁-C₄)-Alkoxycarbonyl und Halogen, substituiert sein können, oder
R¹ für (5- bis 6-gliedriges) Heteroarylamino oder (C₆-C₁₀)Aryl(C₁-C₃)alkylamino steht,
R² für (C₁-C₆)Alkyl, (C₁-C₆)Alkoxy, und Aminocarbonyl steht, oder die oben genannte Bedeutung von R¹ aufweisen kann und von dieser Bedeutung gleich oder verschieden sein kann, oder
R¹ und R² gemeinsam mit dem Stickstoffatom, an das sie gebunden sind einen gesättigten oder ungesättigten Ring mit 4 bis 14 Kohlenstoffatomen bilden, dessen Ringkohlenstoffatome gegebenenfalls durch 1 bis 4 Reste ausgewählt aus der Gruppe, die aus O, S, S=O, C=O und N-R⁸ besteht, ersetzt sein können, und wobei der Ring gegebenenfalls durch 1 bis 3 Substituenten substituiert sein kann, die aus der Gruppe ausgewählt werden, die besteht aus Imino, Hydroxy, (C₁-C₃)Alkyl, 1,3-Dioxolan-2,2-diyl, (C₁-C₃)Alkoxycarbonyl, (C₁-C₆)Alkoxycarbonylamino, Benzyl, Phenylamino, Piperidyl, Hydroxy-(C₁-C₃)alkyl, Benzoyl, Aminocarbonyl, (C₁-C₃)Acyl, (C₁-C₆)Alkylaminocarbonyl, Morpholinocarbonyl und Phenylaminocarbonyl besteht,
und worin
R⁸ für Wasserstoff, eine geradkettige oder verzweigtkettige (C₁-C₆)Alkylgruppe, die gegebenenfalls durch 1 bis 2 Substituenten substituiert sein kann, die aus der Gruppe ausgewählt werden, die aus Phenyl, Hydroxy, Halogenphenyl, Pyrrolidinyl und (C₁-C₃)Alkoxycarbonyl besteht, oder
R⁸ für (C₃-C₆)Cycloalkyl oder Phenyl steht, das gegebenenfalls substituiert sein kann mit 1 bis 3 Substituenten, die aus der Gruppe ausgewählt werden, die besteht aus Halogen, Hydroxy, (C₁-C₃)Alkoxy, Nitro und Trifluormethyl, oder
R⁸ für Pyridyl oder (C₁-C₆)Alkoxycarbonyl steht,
R³ für Wasserstoff, Halogen, Hydroxy oder (C₁-C₃)Acyloxy steht,
und
R⁴ und R⁵ jeweils unabhängig für eine (C₁-C₃)Alkylgruppe stehen,
A für einen Naphthalindiylrest oder einen Phenylenrest steht, die gegebenenfalls durch eine (C₁-C₃)Alkylgruppe, Halogen oder Hydroxy substituiert sein können, und
D und E für Wasserstoff stehen,
sowie deren pharmazeutisch verträglichen Salze.

3. Verbindungen der allgemeinen Formel (I) nach Anspruch 1 oder 2, worin
R¹ für Wasserstoff, Cyclopropyl, (C₂-C₆)Alkenyl, (C₁-C₆)Alkoxy(C₁-C₆)alkyl oder eine geradkettige oder verzweigtkettige (C₁-C₁₂)-Alkylgruppe steht, die gegebenenfalls durch 1 bis 3 Substituenten substituiert sein kann, die aus der Gruppe ausgewählt werden die aus Hydroxy, Cyano, -N(R⁶)R⁷, Phenyl, das gegebenenfalls durch (C₁-C₃)Alkoxy substituiert sein kann, Pyridyl, das gegebenenfalls durch (C₁-C₃)Alkyl substituiert sein kann, Furyl, Indolyl und (C₁-C₄)Alkoxycarbonyl besteht,
worin
R⁶ und R⁷ gleich oder verschieden sein können und ausgewählt werden aus Wasserstoff, (C₁-C₆)Alkoxycarbonyl und (C₁-C₃)Alkyl, das gegebenenfalls durch Di(C₁-C₃)Alkylamino substituiert sein kann, oder
R⁶ und R⁷ gemeinsam mit dem Stickstoffatom, an das sie gebunden sind Morpholino oder Piperidino bilden, das gegebenenfalls durch 1 bis 2 (C₁-C₃)Alkylgruppen substituiert sein kann,
oder
R¹ für Phenyl steht, das gegebenenfalls durch 1 bis 3 Substituenten substituiert sein kann, die ausgewählt werden aus der Gruppe die aus Hydroxy, (C₁-C₄)Alkoxycarbonyl, Halogen und (C₁-C₃)Alkyl besteht, oder für Isochinolinyl, Pyridyl, das gegebenenfalls durch (C₁-C₃)Alkyl oder Amino substituiert sein kann, Pyrimidinyl, das gegebenenfalls durch Hydroxy substituiert sein kann, Thiazolyl, Pyrazolyl oder für Naphthyridinyl steht, oder
R¹ für Pyridylamino oder Benzylamino steht, und
R² die gleiche Bedeutung wie die von R¹, wie zuvor definiert, aufweisen kann, und mit dieser Bedeutung gleich oder verschieden sein kann oder
R² für (C₁-C₃)Alkoxy oder Aminocarbonyl stehen kann,
oder
R¹ und R² gemeinsam mit dem Stickstoffatom, an das sie gebunden sind, einen Ring bilden, der ausgewählt wird aus der Gruppe die aus Morpholino, das gegebenenfalls durch 1 bis 3 (C₁-C₃)Alkylgruppen substituiert sein kann,
Thiomorpholino, das gegebenenfalls ein Sauerstoffatom am Schwefel fragen kann,
Thiazolidinyl,
1-Azacyclopent-3-en-1-yl,
1-Aza-4,7,10,13-tetraoxacyclopentadecan-1-yl,
einem 5- bis 8-gliedrigen gesättigten Ring, in dem gegebenenfalls ein Ringkohlenstoffatom durch eine Gruppe der Formel -NR⁸ ersetzt sein kann, worin
R⁸ ausgewählt wird aus Wasserstoff, gegebenfalls durch 1 bis 3 Substituenten ausgewählt aus Halogen, Hydroxy, (C₁-C₃)Alkoxy, Trifluormethyl und/oder Nitro, substituiertem Phenyl, Halogenphenyl, gegebenfalls durch 1 bis 2 Halogenphenylgruppen, Phenyl und/oder Pyrrolidinyl substituiertem (C₁-C₃)Alkyl, Cyclopropyl, Hydroxy(C₁-C₃)alkyl, Pyridyl und (C₁-C₄)Alkoxycarbonyl,
und der gegebenfalls durch 1 bis 2 Substituenten ausgewählt aus (C₁-C₃)Alkyl, Hydroxy(C₁-C₃)alkyl, Hydroxy, 1,3-Dioxolan-2,2-diyl, Oxo, (C₁-C₄)Alkoxycarbonyl, (C₁-C₄)Alkoxycarbonylamino, Benzyl, Phenylamino, Piperidino, Benzoyl, Aminocarbonyl, (C₁-C₃)-Acyl, (C₁-C₆)Alkylaminocarbonyl, Morpholinocarbonyl und Phenylaminocarbonyl substituiert sein kann,
und einem Rest der Formel: 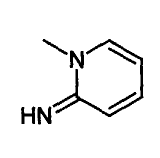 besteht,
R³ für Wasserstoff, Chlor, Fluor, Acetyloxy, Hydroxy steht,
R⁴ und R⁵ für Methyl stehen,
A für Naphthalin-1,5-diyl, Naphthalin-1,6-diyl oder 1,3-Phenylen steht, das gegebenenfalls durch Hydroxy, Chlor, Fluor oder (C₁-C₃)Alkyl substituiert sein kann, und worin
D und E für Wasserstoff stehen.

4. Verbindungen nach irgendeinem der Ansprüche 1 bis 3 der Formel: 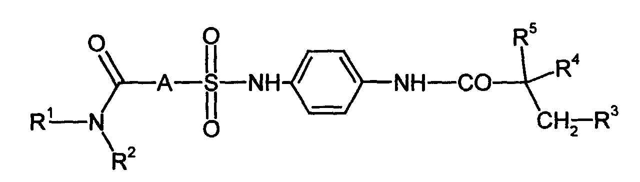 worin R¹,R²,R³,R⁴,R⁵ und A wie oben definiert sind.

5. Verbindungen nach irgendeinem der Ansprüche 1 bis 4, worin A Naphthalindiyl ist.

6. Verbindungen nach irgendeinem der Ansprüche 1 bis 5, worin R¹ Wasserstoff ist und R² ausgewählt wird aus Pyridyl, Phenyl, Hydroxyphenyl oder (C₁-C₄)Alkoxycarbonylphenyl, oder R¹ und R² gemeinsam mit dem Stickstoffatom, an das sie gebunden sind, einen Ring der Formel: 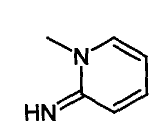 bilden, oder für Thiomorpholino, 4-Oxopiperidino, 4-N-(Cyclopropyl)piperazin-1-yl, Piperidino, 1-Azacyclooctan-1-yl, 1-Azacycloheptan-1-yl oder 1,3-Thiazolidin-1-yl stehen.

7. Verbindungen nach irgendeinem der Ansprüche 1 bis 6, worin
R³ für Wasserstoff oder Halogen steht,
R⁴ und R⁵ jeweils für eine Methylgruppe stehen,
A für einen Phenylen oder Naphthalindiyl-Rest steht, und
D und E für Wasserstoff stehen.

8. Verfahren zur Herstellung von Verbindungen der allgemeinen Formel (I) nach Anspruch 1, dadurch gekennzeichnet, daß man Verbindungen der allgemeinen Formel (II) 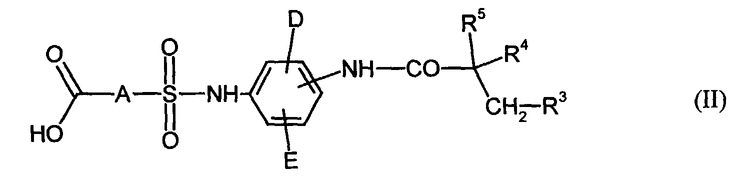
worin die Substituenten die obengenannte Bedeutung aufweisen, gegebenenfalls nach Überführung in das Säurechlorid oder nach Aktivierung nach literaturbekannten Verfahren, mit Aminen der allgemeinen Formel (III) 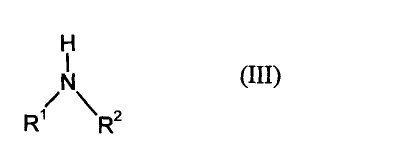 umsetzt, oder
Verbindungen der allgemeinen Formel (IV) 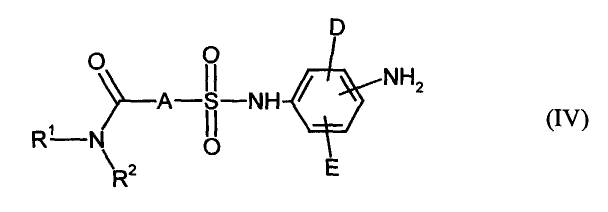
worin die Substituenten die obengenannte Bedeutung besitzen, mit Carbonsäurechloriden der allgemeinen Formel (V) umsetzt 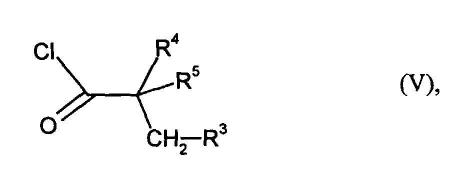
worin die Substituenten die obengenannte Bedeutung besitzen.

9. Verbindungen nach Anspruch 1, zur Verwendung als Arzneimittel.

10. Pharmazeutische Zusammensetzung, die eine Verbindung der allgemeinen Formel (I) nach Anspruch 1 in Mischung mit mindestens einem pharmazeutisch verträglichen Träger oder Exzipienten umfaßt.

11. Verwendung der Verbindung der allgemeinen Formel (I) nach Anspruch 1 zur Herstellung eines Arzneimittels.

12. Verwendung einer Verbindung der allgemeinen Formel (I) nach Anspruch 1 zur Herstellung eines Arzneimittels zur Behandlung von viralen Infektionen, insbesondere Infektionen durch Cytomegalieviren.
